# EUROPEAN PATENT APPLICATION

(11) **EP 4 632 383 A1**
(43) Date of publication of application: **15.10.2025**
(21) Application number: 23900822.0
(22) Date of filing: 25.08.2023
(51) Int. Cl.: G01N 33/574, G01N 33/68

(54) **NOVEL BIOMARKERS FOR DIAGNOSING AND PREDICTING PROGNOSIS OF CHOLANGIOCARCINOMA, AND USE THEREOF**

(30) Priority: 09.12.2022 KR 20220172062; 09.12.2022 KR 20220172070
(71) Applicant: POSTECH Research and Business Development Foundation, Pohang-si, Gyeongsangbuk-do 37673 (KR); CHUNGNAM NATIONAL UNIVERSITY HOSPITAL, Daejeon 35015 (KR)
(72) Inventor: PARK, Seung-Yeol, Pohang-si Gyeongsangbuk-do 37673 (KR); CHOI, Hyewon, Pohang-si Gyeongsangbuk-do 37673 (KR); YEO, Min-Kyung, Daejeon 35249 (KR); KIM, Jin Man, Daejeon 34124 (KR)
(74) Representative: Jung, Minkyu
(86) International application number: PCT/KR2023/012647
(87) International publication number: WO 2024/122800

(57) **Abstract**

The present disclosure relates to novel biomarkers for diagnosing and predicting the prognosis of cholangiocarcinoma, and use thereof. More specifically, the present disclosure provides a method for the diagnosis, prognosis prediction and treatment of cholangiocarcinoma by using, as biomarkers, changes in the level of fucosylation and the sugar chain structure of serum-derived exosomes of cholangiocarcinoma patients. The biomarkers of the present disclosure exhibit excellent specificity and sensitivity for cholangiocarcinoma so as to be used in methods for diagnosis, prognosis prediction and treatment of cholangiocarcinoma patients, and thus can contribute to increasing the treatment efficiency of cholangiocarcinoma patients and reducing mortality from cholangiocarcinoma.

## Description

### [Technical Field]

The present disclosure relates to novel biomarkers for diagnosing and predicting the prognosis of cholangiocarcinoma and use thereof.

### [Background Art]

Cholangiocarcinoma is a malignant tumor that develops from the epithelial cells of the bile duct and is divided into intrahepatic cholangiocarcinoma and extrahepatic cholangiocarcinoma depending on which part of the bile duct it occurs, and used as a concept including gallbladder cancer. Histologically, most of them are adenocarcinomas, and the cause of cholangiocarcinoma has not yet been clearly identified. Cholangiocarcinoma has no symptoms in the early stages, making early diagnosis difficult. There is a case where after cholelithiasis is suspected due to nonspecific symptoms or abnormal liver function levels and a cholecystectomy is performed, cholangiocarcinoma is diagnosed. Recently, with the spread of health checkups, in many cases, cholangiocarcinoma is diagnosed incidentally through abdominal ultrasound examinations. In addition, since the bile duct is closely related to important organs such as the liver and pancreas, cancer may spread to these organs to worsen the prognosis.

Currently, diagnostic methods for the cholangiocarcinoma are combinations of advanced imaging-based diagnostics such as abdominal computed tomography, magnetic resonance imaging, and cholangioscopy; methods of testing blood tumor markers such as carcinoembryonic antigen (CEA), carbohydrate antigen 19-9 (CA19-9), and CA 125; and molecular biochemical tests for evaluating increases in liver function abnormalities such as alkaline phosphatase and gamma-glutamyl transpeptidase. However, there is a limitation that accuracy deteriorates due to each disadvantage. Imaging-based diagnosis has difficulty in distinguishing between wide tumor and non-tumor conditions that simulate cholangiocarcinoma, and blood markers used in molecular biochemical tests are indicators that are not specific to cholangiocarcinoma but may also appear in other solid tumors or infectious diseases, resulting in low accuracy.

Since conventional diagnosis methods for cholangiocarcinoma have limitations, there is a need for a non-invasive method that can accurately diagnose cholangiocarcinoma from the patient's perspective.

Meanwhile, extracellular vesicles (EVs) are very small microvesicles secreted from cells and may be broadly divided into three types based on the size and biological origin: (i) apoptotic bodies with a diameter of 800 nm or more secreted by cells during apoptosis; (ii) microvesicles with a diameter of 100 to 1,000 nm released with the plasma membrane; and (iii) exosomes derived from intracellular endosomes with a diameter of 50 to 100 nm (Ramirez, M.I., et al, Nanoscale, 10(3): p. 881-906. 2018).

The EVs contain various substances such as DNA, mRNA, miRNA, non-coding RNA, and proteins produced in cells, and have various receptors and ligands on their surfaces. It has been confirmed that the composition of these substances varies depending on the type and activity state of the cells secreting the EVs, and the EVs secreted from various cells exist in human body fluids such as blood, ascites, and urine. In particular, EVs released from tumor cells may have unique tumor-specific markers, and changes in EV components secreted from various cells appear depending on changes in the microenvironment around tumor cells. Therefore, the EVs are receiving great attention as potential cancer biomarkers.

To date, traditional research on the biochemical progression of cancer has focused on changes in protein expression, but disease research based on a complex carbohydrate structure, which is a biological component, has not been properly conducted due to difficulties in structural interpretation. However, with the development of technologies such as trace sugar chain structure analysis, sugar chain function analysis, and sugar chain synthesis, the importance of complex carbohydrate chains has been rapidly found, and it has been reported that the development of cancer cells is due to the role of various glycosyltransferases, and that the resulting changes in carbohydrates of glycoproteins are associated with the process of cell carcinogenesis.

Glycosylation is one of the well-known post-translational modification processes. Cancer that develops in a certain organ has unique sugar chains, and sugar chains expressed in glycosphingolipids and glycoproteins are considered to be useful in indicating or inhibiting tumor development. These sugar chains may be not only used for diagnostic purposes, but these sugar chain antigens are also considered excellent targets for immunotherapy in various preclinical studies.

Therefore, since accurate and rapid detection of cholangiocarcinoma is closely associated with the treatment efficacy and survival rate of patients, it is necessary to develop biomarkers based on glycosylation analysis of patient-derived exosomes, as a means for diagnosing and predicting the prognosis of cholangiocarcinoma with high accuracy, which not only reduces the probability of false positives and false negatives in the diagnosis of cholangiocarcinoma, but also does not require additional tests such as unnecessary re-imaging and biopsy.

### [Disclosure]

### [Technical Problem]

Under these situations, the present inventors have made extensive research efforts to develop a method for rapidly and accurately diagnosing cholangiocarcinoma, which is classified as an incurable cancer, with better specificity and sensitivity than conventional biomarkers. As a result, the present inventors discovered that changes in carbohydrate sugar chains of exosomes derived from the blood (serum) of cholangiocarcinoma patients could be applied as an important indicator for monitoring the progression stage of cholangiocarcinoma. Accordingly, when comparing and analyzing the glycation changes of exosomes derived from the serums of normal individuals and cholangiocarcinoma patients, the level of fucosylation increased as alpha 1-3/4 fucose was attached to exosomes (CCA-EV) derived from the serum of cholangiocarcinoma patients, and a peak (sugar chain structure) at a specific GU value of the exosomes of the patients showed a pattern that was distinguished from that of the exosomes of normal individuals, thereby completing the present disclosure by elucidating that early diagnosis and prognosis of cholangiocarcinoma could be accurately and effectively predicted.

Therefore, an object of the present disclosure is to provide a biomarker composition for diagnosing or predicting the prognosis of cholangiocarcinoma, including fucosylation of exosomes; alpha 1-3/4 fucose sugar chains of exosomes; or fucosylation of exosome membrane-bound form, haptoglobin.

Another object of the present disclosure is to provide a composition for diagnosing or predicting the prognosis of cholangiocarcinoma, including an agent capable of detecting fucosylation of exosomes; an agent capable of detecting alpha 1-3/4 fucose sugar chains of exosomes; or an agent capable of detecting fucosylation of exosome membrane-bound form, haptoglobin.

Yet another object of the present disclosure is to provide a kit for diagnosing or predicting the prognosis of cholangiocarcinoma, including the composition.

Still another object of the present disclosure is to provide a method for diagnosing or treating cholangiocarcinoma.

### [Technical Solution]

The terms used herein are used for the purpose of description only, and should not be construed to be limited. A singular expression includes a plural expression unless otherwise defined differently in a context. In the present disclosure, it should be understood that term "including" or "having" indicates that a feature, a number, a step, an operation, a component, a part or the combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which embodiments pertain. Terms which are defined in a generally used dictionary should be interpreted to have the same meaning as the meaning in the context of the related art, and are not interpreted as ideal or excessively formal meanings unless otherwise defined in the present application.

Hereinafter, the present disclosure will be described in detail.

According to one aspect of the present disclosure, the present disclosure provides a biomarker composition for diagnosing or predicting the prognosis of cholangiocarcinoma, including fucosylation of exosomes. Further, the present disclosure provides a composition for diagnosing or predicting the prognosis of cholangiocarcinoma, including an agent capable of detecting fucosylation of exosomes.

The present inventors discovered for the first time that the level of fucosylation in glycosylation of exosomes extracted from the serum isolated from cholangiocarcinoma patients was significantly increased compared to other carcinomas, infectious diseases, and normal conditions, and that when the level of fucosylation was confirmed by extracting exosomes from the serum of cholangiocarcinoma patients before and after surgery, the level of fucosylation in exosomes decreased after surgery, except in cases of recurrence, metastasis, and incomplete removal of cancer.

Accordingly, the present disclosure is characterized in that it is possible to diagnose cholangiocarcinoma, which shows different patterns from other carcinomas, infectious diseases, and normal conditions, and effectively predict its prognosis by using the fucosylation of exosomes in serum that show carbohydrate changes upon occurrence of cholangiocarcinoma as described above as a marker of the present disclosure and detecting the fucosylation using a lectin binding thereto.

That is, the present disclosure may effectively diagnose cholangiocarcinoma by targeting the level of fucosylation, which is specific glycosylation of exosomes in serum, and provide information on the prognosis of patients.

The selection and application of these significant markers may determine the reliability of the results. The significant marker may mean a marker that has high validity because the results obtained by the determination are accurate and has high reliability so that consistent results are shown even when measured repeatedly.

For example, according to an embodiment of the present disclosure, the diagnosis and prognosis prediction marker is a marker having high reliability in which the fucosylation of exosomes in the serum is specifically increased in cholangiocarcinoma. Accordingly, the results determined based on the results obtained by detecting the level of the biomarker of the present disclosure may be reasonably reliable.

The fucosylation of exosomes of the present disclosure may more specifically refer to fucosylation of sugar chains of the exosome surface protein.

In a broad meaning, the term "diagnosis" as used herein means determining the actual condition of a patient's disease in all aspects. The contents of the determination include the name of a disease, the cause of a disease, a type of a disease, severity of a disease, detailed conditions of a disease, the presence or absence of complications, etc.

In the present disclosure, the diagnosis is to determine whether cholangiocarcinoma has developed, the stage of progression, etc.

The term "prognosis prediction" as used herein refers to the action of predicting the course and outcome of a disease in advance. More specifically, the prognosis prediction may be interpreted to mean all actions that the course of the disease after treatment may vary depending on physiological or environmental conditions of the patient, and the course of the disease after treatment is predicted by comprehensively considering the patient's conditions.

The "prognosis" refers to a prediction regarding the progression or recovery of the disease, and refers to a prospect or preliminary assessment. For the purpose of the present disclosure, the prognosis means determining whether treatment has been successful, and whether survival, recurrence, metastasis, drug responsiveness, resistance, and the like have occurred in a subject after cancer treatment. That is, the prognosis refers to the expectation of medical outcome (e.g., possibility of long-term survival, possibility of progression-free survival, disease-free survival rate, etc.), and includes a positive prognosis or a negative prognosis. The negative prognosis includes progression of the disease such as recurrence and drug resistance, or mortality, and the positive prognosis includes remission of a disease such as absence of the disease, and improvement or stabilization of the disease.

For example, in the present disclosure, the prognosis prediction may be interpreted as an action of predicting "progression-free survival (PFS)". The PFS means maintaining the condition without recurrence of cancer during or after treatment of the disease. That is, the predicting of "good prognosis" means that the patient has a high level of progression-free survival to maintain the condition without recurrence, and the predicting of "poor prognosis" means that the patient has a low level of progression-free survival so that the cancer will recur.

For the purpose of the present disclosure, the prognosis prediction means diagnosis and prognosis prediction by confirming the level of the biomarker of the present disclosure in exosomes in patient-derived serum as a biological sample to determine an increase compared to a normal group. In particular, according to the present disclosure, when the level of fucosylation of the sugar chains of the surface protein was confirmed by extracting exosomes after cholangiocarcinoma surgery, the level of fucosylation was still high in cases of recurrence, metastasis, and incomplete removal of cancer. Therefore, the prognosis prediction in the present disclosure means providing information on the prognosis of cholangiocarcinoma by detecting the level of fucosylation of the sugar chains of the surface protein of exosomes after surgery as a biomarker.

In the present disclosure, the exosomes may be obtained using any method known in the art as long as the purpose of the present disclosure can be achieved, and may be easily adjusted and used by those skilled in the art as long as the shape and activity of the exosomes do not exhibit abnormalities.

According to a preferred embodiment of the present disclosure, the agent capable of detecting the fucosylation of exosomes is lectin.

The lectin is a protein that selectively binds to carbohydrates, and is isolated and purified from plants, microorganisms, viruses, invertebrates, and vertebrates, and is widely used as materials for research related to various diseases, and currently, 300 types thereof have been reported.

These lectins are well known to have the function of aggregating and sedimenting red blood cells, and also classify blood types. Recently, the lectins have been reported to have various functions, such as the function of specifically aggregating cancer cells. This is because a specific structure of lectins binds to sugars to enable selective information exchange between cells that have lectin substances and cells that have sugars that bind to the lectin substances.

The lectin as used herein is any lectin that may measure fucosylation as long as the purpose of the present disclosure can be achieved, and for example, may be at least one selected from the group consisting of aleuria aurantia lectin (AAL), aspergillus oryzae lectin (AOL), ulex europaeus agglutinin I (UEA I), and lotus tetragonolobus lectin (LTL), preferably aleuria aurantia lectin (AAL), but is not limited thereto.

As the lectin of the present disclosure, the AAL specifically binds to fucosylated sugar chains of exosomes.

According to an embodiment of the present disclosure, in order to analyze the specific glycation of serum-derived exosomes of cholangiocarcinoma patients, exosomes from the serums of patients with five types of solid cancer (hepatocarcinoma, lung cancer, gastric adenocarcinoma, breast cancer, and prostate cancer) including cholangiocarcinoma and a normal group were extracted, and then three types of glycation (fucosylation, sialylation, and N-acetylglucosamine) in the exosomes were detected using enzyme-linked lectin assay (ELLA). As a result, the level of fucosylation was found to be highest in cholangiocarcinoma. Therefore, the level of fucosylation of serum-derived exosomes of cholangiocarcinoma patients provided excellent sensitivity and specificity that are clearly distinguished from other cancers.

That is, the measurement of fucosylation of exosomes of the present disclosure is useful for diagnosing cholangiocarcinoma.

According to another aspect of the present disclosure, the present disclosure provides a kit for diagnosing or predicting the prognosis of cholangiocarcinoma, including the composition including the agent capable of detecting fucosylation of the above-described exosomes.

The kit of the present disclosure may detect markers by checking the levels of the biomarkers of the present disclosure. The kit of the present disclosure may include not only primers and probes for measuring the expression of markers for diagnosing or predicting the prognosis of cholangiocarcinoma, antibodies that selectively recognize the markers or fragments thereof that retain the antigen-binding ability, but also compositions or devices of one or more other components suitable for the biomarker analysis method.

In addition, the kit of the present disclosure may include an antibody that specifically binds to a marker component, a secondary antibody conjugate to which a label that develops color by reaction with a substrate is conjugated, a chromogenic substrate solution that color-reacts with the label, a washing solution, an enzyme reaction stop solution, etc., and may be manufactured with a plurality of separate packagings or compartments containing reagent components to be used.

Preferably, the kit of the present disclosure provides a kit including a biotin-labeled lectin and a streptavidin-conjugated chromogenic enzyme that bind to exosomes in the serum derived from a target subject.

The principle by which lectin detects changes in fucosylation of a sample within the diagnostic kit is as follows.

That is, when the exosomes extracted from the subject-derived serum are added to the kit, the biotin-labeled lectin binds to the exosomes in which a change in the sugar chain has occurred. Thereafter, the streptavidin-conjugated chromogenic enzyme or fluorescent substance and the chromogenic substrate are sequentially conjugated, and the results are displayed as absorbance or fluorescence. If the binding level between the exosomes and the lectin increases, it may be determined to be cholangiocarcinoma.

In the diagnostic kit according to the present disclosure, the substrate may be selected from the group consisting of a nitrocellulose membrane, a 96-well plate synthesized with a polyvinyl resin, a 96-well plate synthesized with a polystyrene resin, and a glass-made slide glass, but is not limited thereto.

The lectin included in the diagnostic kit according to the present disclosure is a biotin-labeled lectin, but is not limited thereto. In addition, the chromogenic enzyme is selected from the group consisting of horseradish peroxidase (HRP), alkaline phosphatase, and luciferase, and the fluorescent substance may be fluorescein isothiocyanate (FITC) or tetramethylrhodamine isothiocyanate (TRITC), but is not limited thereto.

According to yet another aspect of the present disclosure, the present disclosure provides a method for diagnosing and treating cholangiocarcinoma, including the following steps:
(a) isolating exosomes derived from a subject;
(b) measuring the level of fucosylation of the exosomes by treating the isolated exosomes with an agent capable of detecting fucosylation; and
(c) administering a cholangiocarcinoma therapeutic agent to a subject diagnosed with cholangiocarcinoma in step (b).

According to a preferred embodiment of the present disclosure, when the level of fucosylation of the exosomes in step (b) is increased compared to a normal control sample, it may be diagnosed as cholangiocarcinoma or predicted to have a poor prognosis.

As used herein, the term "subject" means an individual for whom the occurrence of cholangiocarcinoma is to be identified or predicted. The subject may be a vertebrate, specifically a mammal, amphibian, reptile, bird, etc., and more specifically a mammal, for example, a human (*Homo sapiens*)*.*

As used herein, the term "biological sample" means any sample obtained from a subject from whom the biomarker of the present disclosure may be detected.

The biological sample may be at least one selected from the group consisting of blood, serum, plasma, saliva, sputum, and urine, preferably serum, and may be treated and prepared using a method commonly used in the technical field of the present disclosure.

The agent capable of detecting the fucosylation in step (b) is lectin, and the lectin may be labeled with biotin, but is not limited thereto as long as the intended effect of the present disclosure can be achieved.

The measurement in step (b) may be performed through at least one selected from the group consisting of enzyme-linked lectin assay (ELLA), SDS-PAGE, Western blotting, enzymelinked immunosorbent assay (ELISA), immunoprecipitation, and immunofluorescence, and is not limited thereto as long as the intended effect of the present disclosure can be achieved.

The cholangiocarcinoma therapeutic agent may use any anticancer agent known in the art as long as the purpose of the present disclosure can be achieved, and for example, at least one selected from the group consisting of 5-fluorouracil, gemcitabine, cisplatin, capecitabine, oxaliplatin, doxorubicin, carboplatin, cyclophosphamide, ifosfamide, nidran, bleomycin, mitomycin C, cytarabine, trimetrexate, methotrexate, etoposide, vinblastine, vinorelbine, alimta, altretamine, procarbazine, taxol, taxotere, lucitanib, erdafitinib, herceptin, pertuzumab, topotecan, pemigatinib, infigratinib, and irinotecan, but is not limited thereto.

Since the method of the present disclosure utilizes the biomarker for diagnosing or predicting the prognosis of cholangiocarcinoma of the present disclosure described above, duplicated descriptions are omitted to avoid excessive complexity of this specification.

According to yet another aspect of the present disclosure, the present disclosure provides a biomarker composition for diagnosing or predicting the prognosis of cholangiocarcinoma, including alpha 1-3/4 fucose sugar chains of exosomes. Further, the present disclosure provides a composition for diagnosing or predicting the prognosis of cholangiocarcinoma, including an agent capable of detecting alpha 1-3/4 fucose sugar chains of exosomes as an active ingredient. The present disclosure provides a kit for diagnosing or predicting the prognosis of cholangiocarcinoma, including the composition.

In an embodiment of the present disclosure, when the sugar chain structure of GU showing different peaks from that of normal individuals was distinguished to analyze the detailed glycan structure of exosomes (CCA-EV) of cholangiocarcinoma patients, it was confirmed that alpha 1-3/4 fucose in the sugar chain structure of cholangiocarcinoma patient-derived exosomes was significantly increased compared to the sugar chain of normal exosomes. Further, when the glycan structure was designated using glucose units (GU), double and triple antenna structures with neutral glycans (peaks a to c) and sialylated glycans (peaks d to 1) were shown, and interestingly, it was found that peak "k" was higher in normal-EV than in CCA EV, whereas peak "l" was higher in CCA-EV than in normal EV. It was found that the peak k was a tri-sialylated, tri-antennary sugar chain (A3G3S3; Oxford notation name) sugar chain of the following Structural Formula 1, and the peak l was a tri-sialylated, a1-3/4 fucosylated, tri-antennary sugar chain (A3G3FS3; Oxford notation name) sugar chain of the following Structural Formula 2:
tri-sialylated, tri-antennary sugar chain (A3G3S3) of the following Structural Formula 1 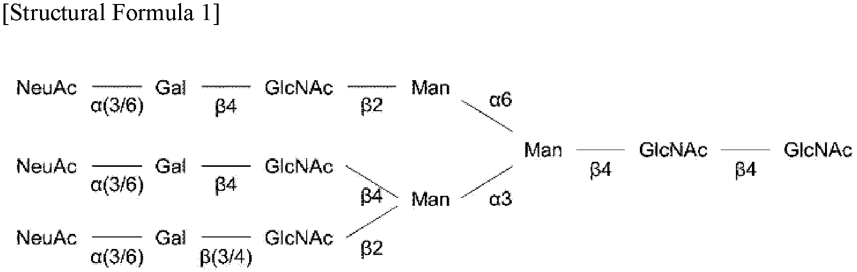 and
tri-sialylated, a1-3/4 fucosylated, tri-antennary sugar chain (A3G3FS3) of the following Structural Formula 2

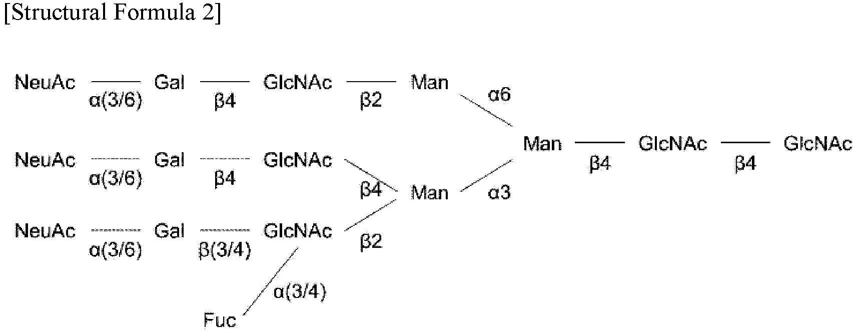

This suggests that these sugar chain structures, as well as the aforementioned alpha 1-3/4 fucosylation, are specific diagnostic markers for cholangiocarcinoma.

In addition, more specifically, the alpha 1-3/4 fucose sugar chain of the exosome of the present disclosure refers to a sugar chain of β-haptoglobin, which is an exosome surface protein.

The present inventors analyzed the sugar chain structure of exosomes extracted from the serum isolated from normal individuals and cholangiocarcinoma patients, and compared the differences, and as a result, discovered for the first time that among the sugar chain structures of exosomes, the alpha 1-3/4 fucose sugar chain was significantly increased in cholangiocarcinoma. In addition, it was confirmed that the surface protein of exosomes with increased alpha 1-3/4 fucose sugar chains was β-haptoglobin.

Accordingly, the present disclosure is characterized in that it is possible to diagnose cholangiocarcinoma, which shows different patterns from normal conditions, and effectively predict its prognosis by using the alpha 1-3/4 fucose sugar chains of exosomes in serum that show sugar chain changes upon occurrence of cholangiocarcinoma as the marker of the present disclosure and treating the alpha 1-3/4 fucose sugar chains with a hydrolase to separate and then detect the alpha 1-3/4 fucose sugar chains.

That is, the present disclosure may effectively diagnose cholangiocarcinoma by targeting the level of alpha 1-3/4 fucose, which is a specific sugar chain of exosomes in the serum, and provide information on the prognosis of patients.

The alpha 1-3/4 fucose sugar chain of the present disclosure may be detected by performing any method used for sugar chain analysis known in the art as long as the purpose of the present disclosure can be achieved, and for example, may be detected by at least one selected from the group consisting of high-performance liquid chromatography (HPLC), capillary electrophoresis, isoelectric focusing, mass spectrometry, porous graphite chromatography, and SDS-PAGE, but is not limited thereto.

Accordingly, the agent capable of detecting the alpha 1-3/4 fucose sugar chain of the present disclosure may include, without limitation, a detection agent known in the art that specifically binds thereto, and may refer to various reagents and agents utilized in at least one method selected from the group consisting of high-performance liquid chromatography (HPLC), capillary electrophoresis, isoelectric focusing, mass spectrometry, porous graphite chromatography, and SDS-PAGE used in the above-described sugar chain analysis.

For detection of the alpha 1-3/4 fucose sugar chains of the present disclosure, sugar chains isolated from the exosomes may be treated with a hydrolase.

The hydrolase that may be used in the present disclosure may be used with any hydrolase known in the art that may be used to measure the alpha 1-3/4 fucose sugar chains as long as the purpose of the present disclosure can be achieved.

For example, the hydrolase may be at least one exoglycosidase selected from the group consisting of sialidase, galactosidase, fucosidase, N-acetylgalactosaminidase, neuraminidase, glucosidase, N-acetylglucosaminidase, and mannosidase.

Preferably, the hydrolase may be a combination of sialidase and galactosidase; a combination of sialidase, galactosidase and fucosidase (e.g., alpha 1-3/4 fucosidase); a combination of sialidase, galactosidase, fucosidase (e.g., alpha 1-3/4 fucosidase) and N-acetylglucosaminidase, but is not limited thereto.

According to an embodiment of the present disclosure, in order to analyze the specific sugar chain structure of serum-derived exosomes of cholangiocarcinoma patients, the exosomes were treated with a specific cleavage enzyme for alpha 1-3/4 fucose, such as alpha 1-3/4 fucosidase, and then HPLC was performed to detect a peak caused by alpha 1-3/4 fucose. As a result, the alpha 1-3/4 fucose sugar chain level was shown to be high in cholangiocarcinoma. Therefore, the alpha 1-3/4 fucose levels in serum-derived exosomes of cholangiocarcinoma patients provided excellent sensitivity and specificity that were clearly distinguished from normal individuals.

That is, the measurement of the alpha 1-3/4 fucose sugar chains of exosomes of the present disclosure is useful for diagnosing cholangiocarcinoma.

According to yet another aspect of the present disclosure, the present disclosure provides a method for diagnosing and treating cholangiocarcinoma, including the following steps:
(a) isolating sugar chains from subject-derived exosomes;
(b) measuring the level of alpha 1-3/4 fucose sugar chains among the isolated exosome-derived sugar chains; and
(c) administering a cholangiocarcinoma therapeutic agent to a subject diagnosed with cholangiocarcinoma in step (b).

According to a preferred embodiment of the present disclosure, when the level of alpha 1-3/4 fucose among the exosome-derived sugar chain structures of step (b) is increased compared to a normal control sample, it may be determined as cholangiocarcinoma or predicted to have a poor prognosis.

Since the method of the present disclosure uses the biomarker for diagnosing or predicting the prognosis of cholangiocarcinoma of the present disclosure described above, duplicated descriptions are omitted to avoid excessive complexity of this specification.

According to yet another aspect of the present disclosure, the present disclosure provides a composition for diagnosing or predicting the prognosis of cholangiocarcinoma, including an agent for detecting fucosylation of an exosome membrane-bound form, haptoglobin, and a kit for diagnosing or predicting the prognosis of cholangiocarcinoma, including the composition.

The present inventors have first found that while fucosylation of soluble haptoglobin makes it difficult to diagnose cholangiocarcinoma, the haptoglobin in a form bound to an exosome membrane extracted from serum isolated from cholangiocarcinoma patients can not only diagnose cholangiocarcinoma, but also diagnose cholangiocarcinoma depending on the degree of its fucosylation.

Accordingly, the present disclosure may effectively diagnose cholangiocarcinoma by detecting fucosylation of haptoglobin in the exosome membrane-bound form in the serum, which indicates a change in carbohydrates when cholangiocarcinoma occurs as described above, using the marker of the present disclosure as a target, and can provide information on the prognosis of the patients.

According to yet another aspect of the present disclosure, the present disclosure provides a method for diagnosing and treating cholangiocarcinoma, including: (a) isolating sugar chains from subject-derived exosomes; (b) detecting tri-sialylated, tri-antennary sugar chain (A3G3S3) of the following Structural Formula 1; or tri-sialylated, a1-3/4 fucosylated, tri-antennary sugar chain (A3G3FS3) of the following Structural Formula 2 among the isolated sugar chains of the exosomes; and (c) administering a cholangiocarcinoma therapeutic agent to a subject diagnosed with cholangiocarcinoma in step (b).

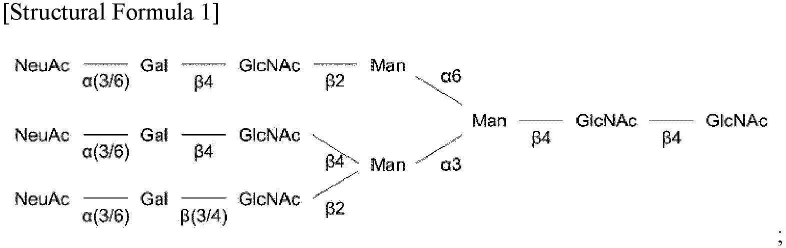

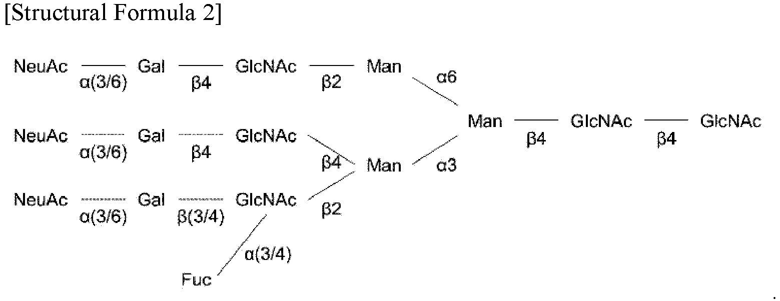

The sugar chains refer to exosome surface protein sugar chains.

The exosome sugar chains of step (b) are detected through glucose unit (GU) by high-performance liquid chromatography (HPLC). As the glucose unit analysis result, it is characterized that when there is a significant difference between normal exosomes and cholangiocarcinoma exosomes by comparing at least one of the expression frequency and expression level of the peak corresponding to a specific GU value, the sugar chain structure has been analyzed based on the peak and selected as a cholangiocarcinoma diagnostic marker.

According to an embodiment of the present disclosure, a peak in the glucose unit 10-11 was significantly higher in normal exosome control samples than in cholangiocarcinoma exosome patient samples. Based on this, when the peak in the glucose unit 10-11 is lower than that of a normal sample, it may be determined as cholangiocarcinoma or to have a poor prognosis.

More specifically, according to a preferred embodiment of the present disclosure, when a sugar chain corresponding to the peak in the glucose unit 10-11 was confirmed, the sugar chain had a tri-sialylated, tri-antennary sugar chain (A3G3S3; Oxford notation name) sugar chain structure.

Accordingly, when the tri-sialylated, tri-antennary sugar chain (A3G3S3) sugar chain in step (b) is reduced compared to the normal control sample, it may be determined as cholangiocarcinoma or to have a poor prognosis.

In addition, according to an embodiment of the present disclosure, a peak in the glucose unit 11-12 was significantly higher in cholangiocarcinoma exosome patient samples than in normal exosome control samples. Based on this, when the peak in the glucose unit 11-12 is higher than that of a normal sample, it may be determined as cholangiocarcinoma or to have a poor prognosis.

More specifically, according to a preferred embodiment of the present disclosure, when a sugar chain corresponding to the peak in the glucose unit 11-12 was confirmed, the sugar chain had a tri-sialylated, a1-3/4 fucosylated, tri-antennary sugar chain (A3G3FS3; Oxford notation name) sugar chain structure.

Accordingly, when the tri-sialylated, a1-3/4 fucosylated, tri-antennary sugar chain (A3G3FS3) sugar chain in step (b) is increased compared to the normal control sample, it may be determined as cholangiocarcinoma or to have a poor prognosis.

In order to measure the level of a sugar chain, which is a cholangiocarcinoma marker of the present disclosure, it is necessary to cut off the sugar chain contained in the sample from the protein to which the sugar chain is bound, and any method known in the art may be used as a method for cutting off the sugar chain from the protein.

In addition, the method for detecting a sugar chain and measuring the content of the sugar chain, for analyzing the sugar chain as a cholangiocarcinoma marker of the present disclosure, is not particularly limited as long as the method is a method capable of detecting and measuring the marker sugar chain of the present disclosure, but may include normal-phase or reverse-phase high-performance liquid chromatography, mass spectrometry, nuclear magnetic resonance, and a method using the sugar chain-specific antibody or lectin of the present disclosure.

When the present inventors analyzed the N-glycosylation sugar chain structure of exosomes extracted from the serum isolated from cholangiocarcinoma patients, the present inventors discovered for the first time that peaks at specific glucose unit values were significantly different from those of normal individuals.

Accordingly, the present disclosure is characterized in that it is possible to diagnose cholangiocarcinoma, which shows different patterns from normal conditions, and effectively predict its prognosis by using the glucose unit values of exosomes in the serum that show sugar chain changes upon occurrence of cholangiocarcinoma as a marker of the present disclosure and comparing the sugar chain structure of the peak corresponding thereto.

That is, the present disclosure may effectively diagnose cholangiocarcinoma by targeting a specific sugar chain of exosomes in the serum, and provide information on the prognosis of patients.

The selection and application of these significant markers may determine the reliability of the results. The significant marker may mean a marker that has high validity because the results obtained by the determination are accurate and has high reliability so that consistent results are shown even when measured repeatedly.

For example, according to an embodiment of the present disclosure, the diagnosis and prognosis prediction marker is a marker having high reliability in which the sugar chain structure of exosomes in the serum is specifically increased in cholangiocarcinoma. Accordingly, the results determined based on the results obtained by detecting the level of the biomarker of the present disclosure may be reasonably reliable.

The sugar chains of the exosomes of the present disclosure may more specifically refer to sugar chains of the exosome surface protein.

For the purpose of the present disclosure, the diagnosis and the prognosis prediction mean diagnosis and prognosis prediction by confirming the level of the biomarker of the present disclosure in exosomes in patient-derived serum as a biological sample to determine a significant difference compared to a normal group.

In addition, according to yet another aspect of the present disclosure, there are provided a biomarker composition for diagnosing or predicting the prognosis of cholangiocarcinoma, including any one of the following sugar chains of exosomes derived by a difference in glucose unit peaks as described above; a composition for diagnosing or predicting the prognosis of cholangiocarcinoma, including an agent capable of detecting any one of the above-described sugar chains; and a kit for diagnosing or predicting the prognosis of cancer, including the composition described above:
tri-sialylated, tri-antennary sugar chain (A3G3S3) sugar chain of the following Structural Formula 1 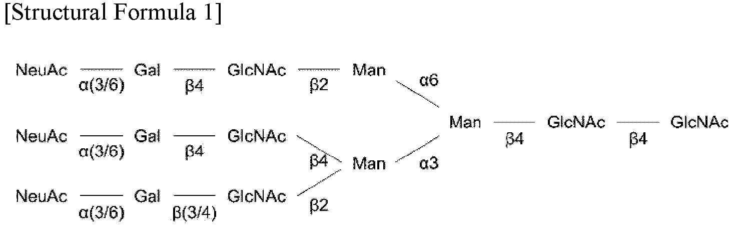 or
tri-sialylated, a1-3/4 fucosylated, tri-antennary sugar chain (A3G3FS3) sugar chain of the following Structural Formula 2

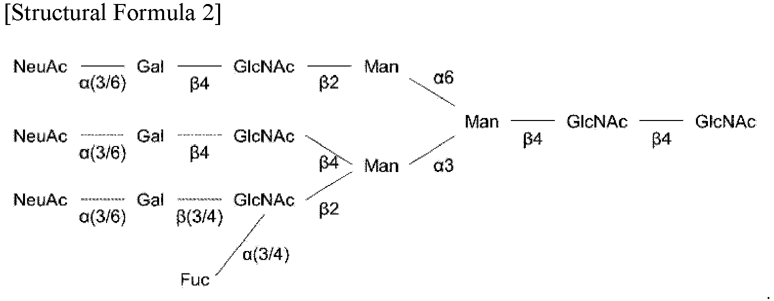

Since the composition and the kit of the present disclosure use the biomarker for diagnosing or predicting the prognosis of cholangiocarcinoma of the present disclosure described above, related duplicated contents are omitted to avoid excessive complexity of this specification.

### [Advantageous Effects]

According to the present disclosure, it is possible to provide a method for diagnosing and predicting the prognosis of cholangiocarcinoma using the level of fucosylation of serum-derived exosomes of cholangiocarcinoma patients as a biomarker, preferably the level of alpha 1-3/4 fucosylation of an exosome membrane-bound form, haptoglobin, and changes in the sugar chain structure. The biomarkers of the present disclosure exhibit excellent specificity and sensitivity for cholangiocarcinoma so as to be used in diagnosis and prognosis prediction of cholangiocarcinoma patients, and thus can contribute to increasing the treatment efficiency of cholangiocarcinoma patients and reducing mortality from cholangiocarcinoma.

### [Description of Drawings]

FIG. 1 shows results of confirming the presence or absence of exosome markers and cell organelle markers in exosomes extracted from serum using centrifugation (UC) and Exoquick (EQ) products.
FIG. 2 shows results of confirming the shapes of exosomes extracted from serum using centrifugation (UC) and Exoquick (EQ) products by electron microscopy and results of confirming the average size of exosomes using NTA.
FIG. 3 shows results of evaluating the level of fucosylation of exosomes extracted from 28 normal sera and 42 cholangiocarcinoma patient sera using AAL lectin.
FIG. 4 shows results of comparing the levels of fucosylation of exosomes of a normal group, a cholangiocarcinoma stage I group, and a cholangiocarcinoma stage II-IV group.
FIG. 5 shows results of comparing the levels of fucosylation of exosomes of a normal group, an intrahepatic cholangiocarcinoma (IHBD) group, and an extrahepatic cholangiocarcinoma (EHBD) group.
FIG. 6 shows results of comparing the levels of fucosylation of exosomes extracted from the blood of cholangiocarcinoma patients before and after surgery.
FIG. 7 shows results of comparing the levels of fucosylation of exosomes in normal, cholecystitis, cholangitis, and cholangiocarcinoma (CCA) groups.
FIG. 8 shows results of comparing the levels of fucosylation of exosomes in normal, hepatocarcinoma, cholangiocarcinoma, lung cancer, gastric adenocarcinoma, breast cancer, and prostate cancer patient groups.
FIG. 9 shows results of comparing the levels of sialic acid of exosomes in normal, hepatocarcinoma, cholangiocarcinoma, lung cancer, gastric adenocarcinoma, breast cancer, and prostate cancer patient groups.
FIG. 10 shows results of comparing the levels of sialic acid and N-acetylglucosamine of exosomes in normal, hepatocarcinoma, cholangiocarcinoma, lung cancer, gastric adenocarcinoma, breast cancer, and prostate cancer patient groups.
FIG. 11 shows HPLC peak profiles of sugar chains isolated from exosomes in the bloods of normal persons and cholangiocarcinoma patients.
FIGS. 12A and 12B show HPLC peak profiles confirmed after treating the sugar chains isolated from exosomes from a normal person (A) and a cholangiocarcinoma patient (B) with glycosidase. Arrows track newly formed peaks as sugar chains are degraded. FIG. 12C shows a sugar chain structure corresponding to each peak. ■: N-acetylglucosamine, •: mannose, o: galactose, ▲: fucose, ◆: sialic acid.
FIGS. 13A and 13B show mass spectrometry of fucosylated proteins in cholangiocarcinoma exosomes (CCA-EVs). A, Representative mass spectrometry chromatogram. The identified peptide matches a sequence of β-Hp. B, Western blot for evaluating the amount of β-Hp in EVs isolated from normal individuals and CCA patients.

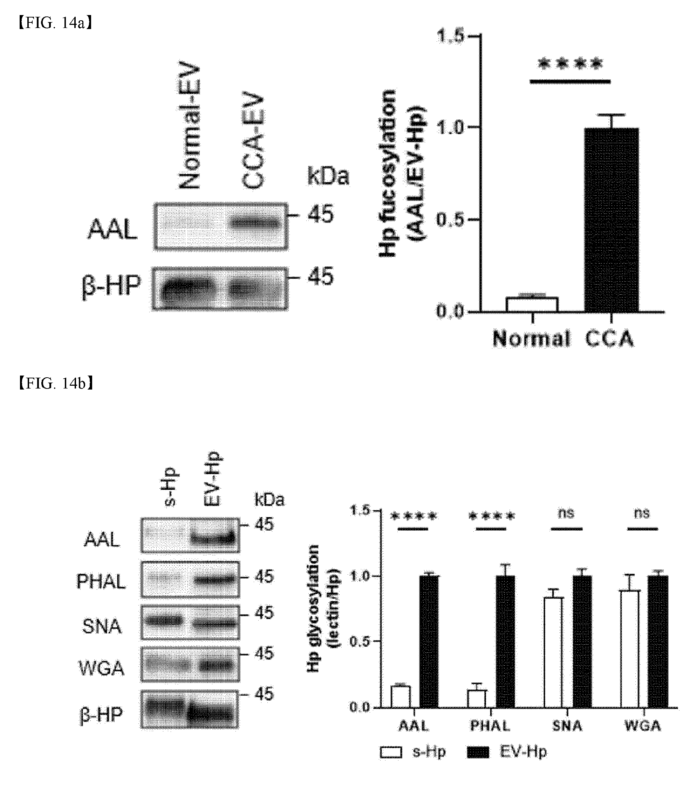

FIGS. 14A to 14G show that β-haptoglobin (β-Hp) is secreted from cells via EVs. A, Aleuria aurantia lectin (AAL) blotting for β-Hp purified from EVs. n = 6. B, Lectin blotting for soluble Hp (s-Hp) and membrane-bound Hp (EV-Hp). n = 3. C, Western blotting for GFP-tagged β-Hp secreted from cells. n = 5. D, *In vitro* transport assay of evaluating β-Hp transport from the ER to the Golgi. Scale bar = 10 µm. E, Western blot for evaluating secretory pathway of transporting β-Hp in cells treated with brefeldin-A (BFA) and chloroquine. n = 4. F, Colocalization of β-Hp with endosomal markers (Rab5 and Rab11) and CD63 assessed by confocal microscopy using Airyscan. Scale bar = 10 µm. Line scans along inserted dotted lines are shown on the right. G, Western blot for evaluating effect of KFERQ-like motif on β-Hp secretion via EV, n = 3. Quantitative data are represented as mean ± SEM. Statistical analysis was performed using a two-tailed Student's t-test. **** p < 0.0001, ns p > 0.05.
FIGS. 15A and 15B show collection of β-haptoglobin (β-Hp) to the extracellular vesicle (EV) membrane. A, Schematic diagram of EV recruitment analysis. B, Western blot for evaluating β-Hp collection to EV membrane, n = 4.

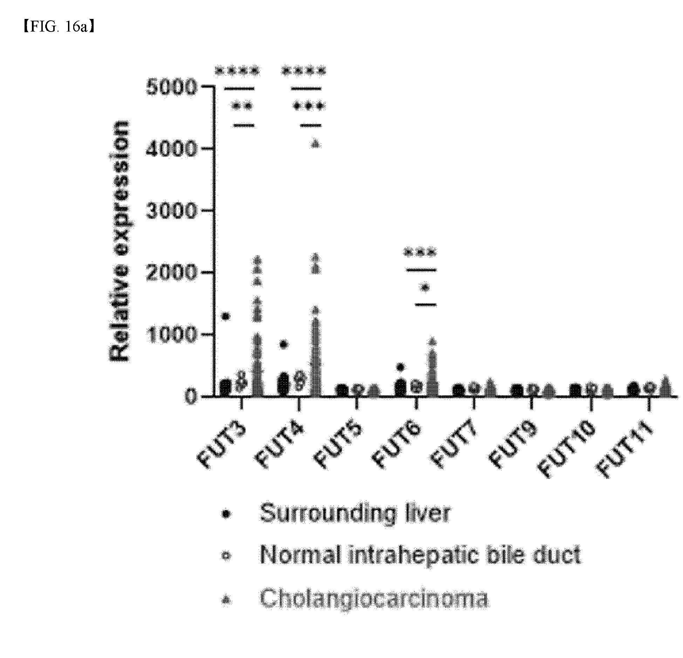

FIGS. 16A to 16G show increased expression of fucosylation-related genes in cholangiocarcinoma (CCA) cells. A, Expression profile of fucosyltransferase (FUT). B, Immunohistochemical staining for FUT-3 and FUT-4. Scale bar = 50 µm. The quantity is shown on the right. C, Immunohistochemical staining for β-haptoglobin (β-Hp). Scale bar = 50 µm. D, qPCR for evaluating the expression levels of FUT-3 and FUT-4 in SNU-1079 and SNU-245 cells, n = 4. E, Western blotting of SNU-1079 and SNU-245 EVs. n = 4. F, Effect of CCA EV on the growth of SNU-1079 cells, n = 3. G, Effect of fucosylated soluble-Hp on cell growth, n = 3. Quantitative data are represented as mean ± SEM. Statistical analysis was performed using a two-tailed Student's t-test. **** p < 0.0001, *** p < 0.001, ** p < 0.01, * p < 0.05, ns p > 0.05.

### [Modes]

However, the following Examples are merely illustrative of the contents of the present disclosure, and the scope of the present disclosure is not limited to the following Examples. Examples of the present disclosure will be provided for more completely explaining the present disclosure to those skilled in the art.

### Experimental method

### Preparation of clinical samples

28 normal sera, 42 sera from cholangiocarcinoma patients, 20 sera from patients with infectious diseases, and 5 sera each from patients with hepatocarcinoma, gastric adenocarcinoma, lung cancer, breast cancer, and prostate cancer were provided by the Chungnam National University Hospital Biobank with the patients' consent.

### Extraction of exosomes from clinical samples

Two methods were used to isolate exosomes from the serum:
As a first method, centrifugation was used. At this time, the serum was diluted with phosphate buffer saline (PBS), centrifuged at 4°C, 2,000 g for 30 minutes, and then only the supernatant was transferred to a new container to remove various cell debris in the serum. Thereafter, the supernatant was centrifuged at 4°C and 200,000 g for 2 hours using an ultracentrifugation container and a dedicated ultracentrifugation rotor (Beckman Coulter). The precipitate formed at this time was exosomes, and the supernatant was removed, and the precipitate was resuspended in PBS to extract exosomes.

As a second method, an Exoquick (SBI system biosciences) product was used. The serum was centrifuged at 1,700 g, 4°C, for 30 minutes, and only the supernatant was collected and mixed with the Exoquick product. At this time, the ratio of serum supernatant : Exoquick was used as 5 : 1. Then, the mixture was reacted on a rotor at 4°C for 18 hours and centrifuged at 1,500 g, 4°C for 30 minutes, and then the supernatant was discarded, and the precipitate was resuspended in PBS to extract exosomes.

### Analysis of exosome glycosylation using lectin

In a step of analyzing the carbohydrates of exosomes using lectin, the present inventors used biotin-bound lectin and detected the exosome-lectin reaction signals through Western blot. First, a 10% acrylamide gel was made and 2 µg to 10 µg of exosomes were loaded. Subsequently, exosome proteins were immobilized onto PVDF membranes via Western blotting, and blocked with 0.9 mM CaCl₂, 0.5 mM MgSO₄, 0.1 mM MnCl₂, 0.1% Tween 20 + Tris buffered saline (TBST+) containing 3% bovine serum albumin (BSA) for two hours at room temperature. Thereafter, the biotin-bound lectin was diluted in TBST+ at a ratio of 1 : 500 to 1 : 1000 and reacted with the membrane at 4°C for 18 hours. At this time, lectins used included AAL, SNA, and WGA. Exosome-lectin reactions were detected for lectin signals using HRP-bound streptavidin. The total protein amount was confirmed by staining with a coomassie solution.

### Statistical analysis

The amount of exosome-lectin reaction detected with HRP-streptavidin and the total amount of proteins stained with coomassie were quantified by the intensity of the detected signal. The intensity was measured using Image J, and the lectin reaction amounts of exosomes from normal serum and exosomes from cholangiocarcinoma patients were divided into 'lectin intensity/total protein amount' and compared. Statistical significance of differences between the groups was analyzed using two-tailed Student's t-test and one-way ANOVA test. The P values below 0.05 were determined to be statistically significant, and the level of significance was expressed as '*'. '*' means the p value of 0.05 or less, '**' means the p value of 0.01 or less, '***' means the p value of 0.001 or less, and '****' means the p value of 0.0001 or less.

### Isolation of sugar chains from exosomes

Exosomes reacted with PNGase F as glycosidase at 37°C for 24 hours to isolate and extract sugar chains attached to exosome surface proteins. The isolated sugar chains passed through a 0.1 µm PTFE filter to remove impurities and then freeze-dried. Next, the sugar chains were dissolved in a 1% formic acid solution to reduce a sugar chain structure and then freeze-died again. Next, the mixture was dissolved in a solution containing a reducing substance, sodium cyanoborohydride (NaBH₃CN) and a fluorescent substance, aminobenzamide (2-AB) and reacted at 65°C for 3 hours. Thereafter, excess 2-AB was removed using a glycan purification kit (Ludger) and the sample was stored at -20°C.

### Confirmation of sugar chain type and amount using high-performance liquid chromatography (HPLC)

Sugar chains extracted from exosomes were isolated and detected using a hydrophilic column (HILIC column, Waters), an HPLC separator (Waters), and a fluorescence detector (Waters). At this time, the solution used in a mobile phase started from a composition of 25% 50 mM ammonium formate : 75% acetonitrile (v/v), and the sugar chains were isolated while constantly increasing a ratio of 50 mM ammonium formate to 45%. All isolation processes were performed at 60°C in the column. Thereafter, the time points at which peaks of sugar chains extracted from exosomes of normal individuals were shown and the sizes (amounts of sugar chains) of the peaks were compared with those of sugar chains extracted from exosomes of cholangiocarcinoma patients.

### Analysis of sugar chain structure using exoglycosidase and HPLC

To confirm the previously analyzed sugar chain structure, the sugar chains were degraded by treatment with various types of glycosidases. The glycosidases used at this time were sialidase (enzyme for degrading sialic acid), galactosidase (enzyme for degrading galactose), N-acetylglucosaminidase (enzyme for degrading N-acetylgucosamine), and fucosidase (enzyme for degrading fucose). The sugar chain samples were treated in combinations of one, two, or three or more of these enzymes, and then reacted at 37°C for 48 hours and applied to an HPLC separator. Thereafter, peaks that disappeared and newly detected peaks when treated with hydrolase were analyzed according to glucose units.

### Example 1. Extraction and testing of exosomes in serum

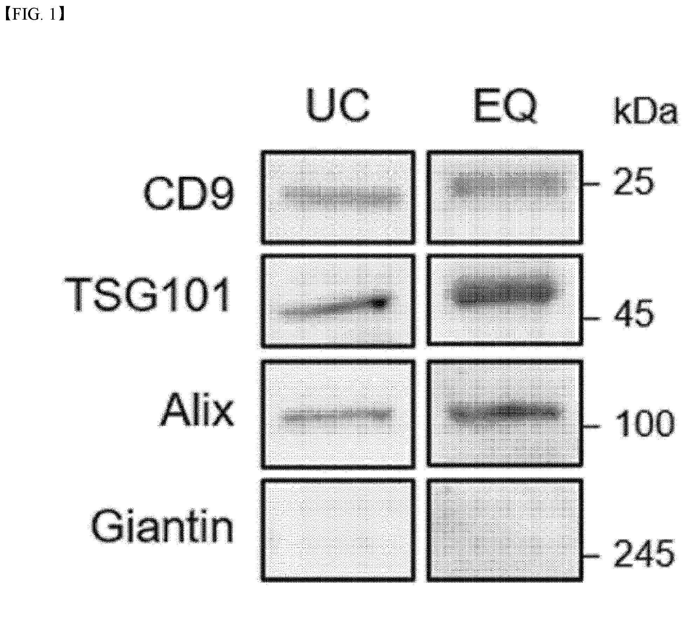

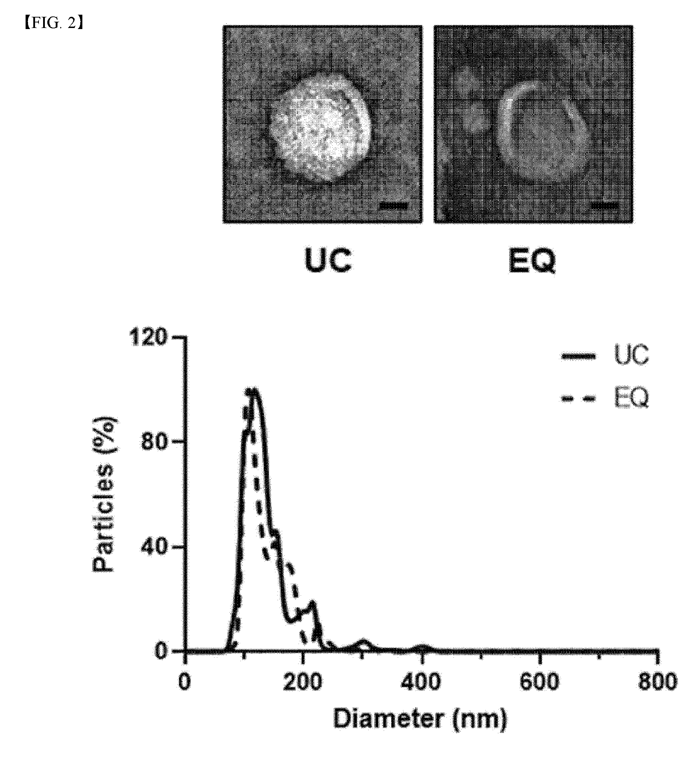

The present inventors extracted exosomes from serum using centrifugation (UC) or Exoquick (EQ) products and identified the exosomes using exosome markers such as CD9, TSG101, and Alix, and intracellular organelle markers such as giantin (FIG. 1). In addition, the average size of exosomes was confirmed using electron microscopy and nanoparticle tracking analysis (NTA) experiments (FIG. 2). These results mean that the biochemical characteristics of exosomes obtained through the two methods are identical. That is, even if any exosome extraction method known in the art is used in addition to the two methods, the biochemical characteristics of the exosome itself do not change, and thus exosomes having the same biochemical characteristics may be obtained. Accordingly, in Examples below, exosomes extracted from the serum using the Exoquick product were used.

### Example 2. Carbohydrate analysis of patient serum-derived exosomes using lectin

The present inventors considered the glycosylation of exosomes derived from the serum of patients with cholangiocarcinoma as a biomarker for the diagnosis of cholangiocarcinoma, and analyzed the glycosylation level of exosomes in the serum using lectin, in order to demonstrate that the change in glycosylation of exosomes in the serum of cholangiocarcinoma patients may be applied as an important indicator for monitoring the progression stage of cholangiocarcinoma.

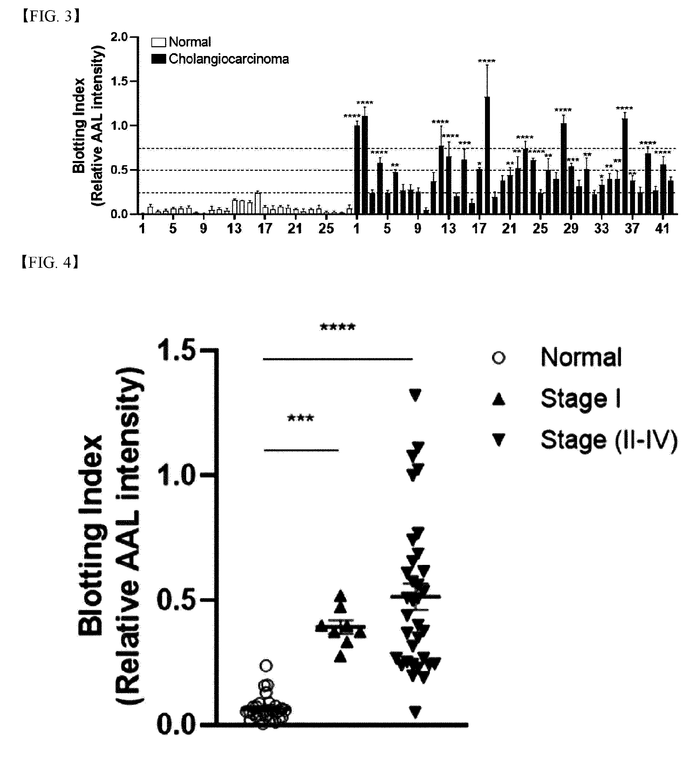

Briefly, AAL lectin reacted with exosomes in the serum isolated from subjects, and then the level of fucosylations of exosomes from cholangiocarcinoma patients and exosomes from normal patients were evaluated through statistical analysis (FIG. 3).

Next, the patients were divided and compared into three groups: normal people, stage I cholangiocarcinoma patients, and stage II-IV cholangiocarcinoma patients by dividing stage I and stage II-IV according to the progression stage (severity) of cancer (FIG. 4).

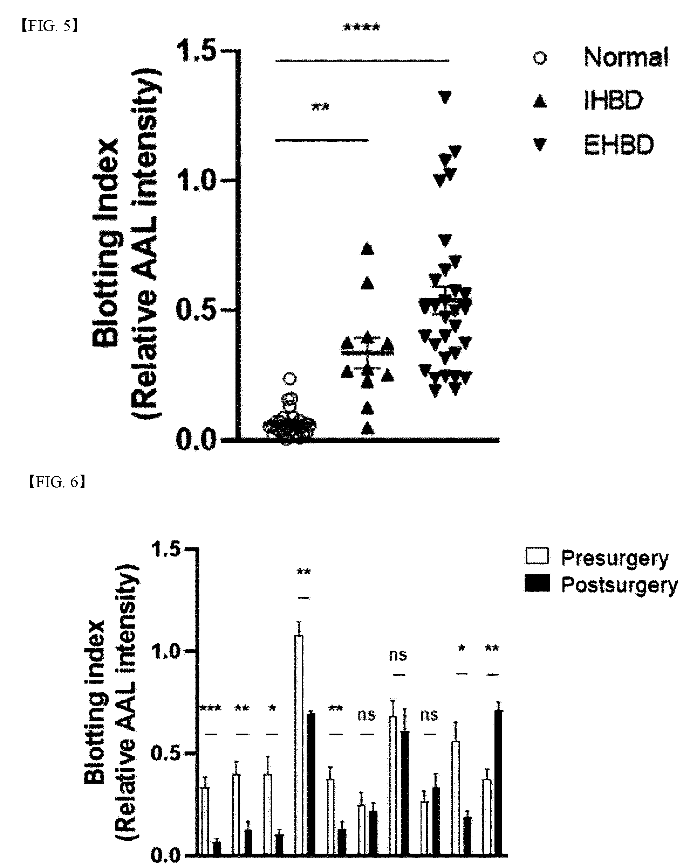

In addition, the cancer was divided into intrahepatic cholangiocarcinoma (IHBD) and extrahepatic cholangiocarcinoma (EHBD) based on a location of cancer onset, and compared with a normal group (FIG. 5).

As a result, as shown in FIGS. 3 to 5, the overall level of fucosylation in the exosomes of cholangiocarcinoma patients was increased compared to normal, and the level of fucosylation increased as the cancer progression stage (severity) worsened, and the level of fucosylation of extrahepatic cholangiocarcinoma patients was confirmed to be increased compared to that of intrahepatic cholangiocarcinoma patients.

In addition, the present inventors confirmed the level of fucosylation by extracting the exosoems from the serum of cholangiocarcinoma patients before and after surgery.

As a result, as shown in FIG. 6, there was no difference in the level of fucosylation in patients with cancer recurrence or metastasis, and the level of fucosylation in exosomes after surgery decreased, except for an increase in the level of fucosylation after surgery in cases of incomplete cancer removal.

In addition, the present inventors extracted exosomes from patients with cholecystitis and cholangitis, which are infectious diseases with similar onset locations, and evaluated the level of fucosylation of the exosomes.

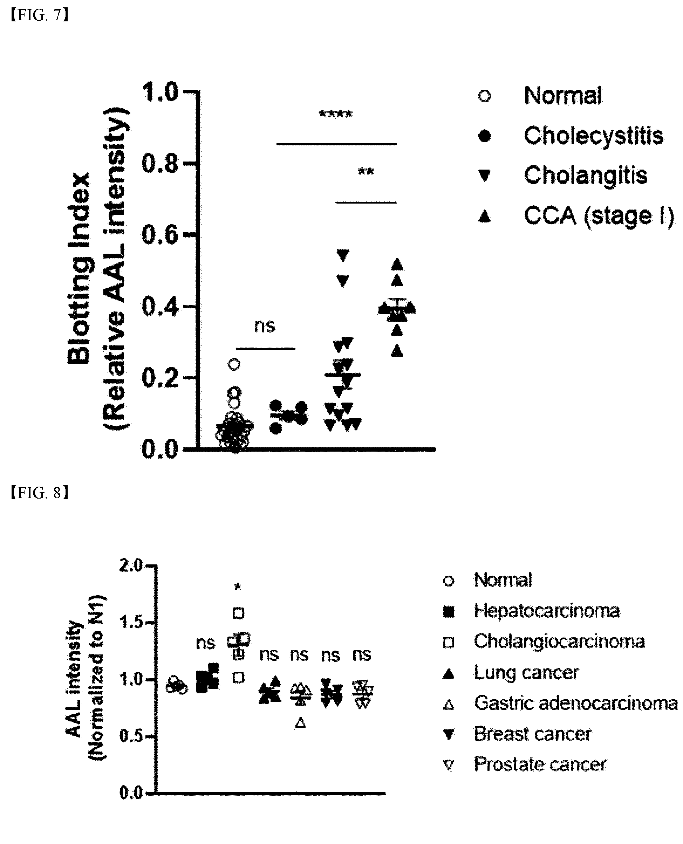

As a result, as shown in FIG. 7, it was confirmed that stage I cholangiocarcinoma patients had a higher level of fucosylation of exosomes than the level of fucosylation of exosomes of patients with infectious diseases.

In addition, exosomes from the sera of patients with five other solid tumors, excluding cholangiocarcinoma, were extracted and carbohydrates were evaluated and compared. Lectins were used with AAL, SNA, and WGA, and carbohydrates were evaluated through statistical analysis.

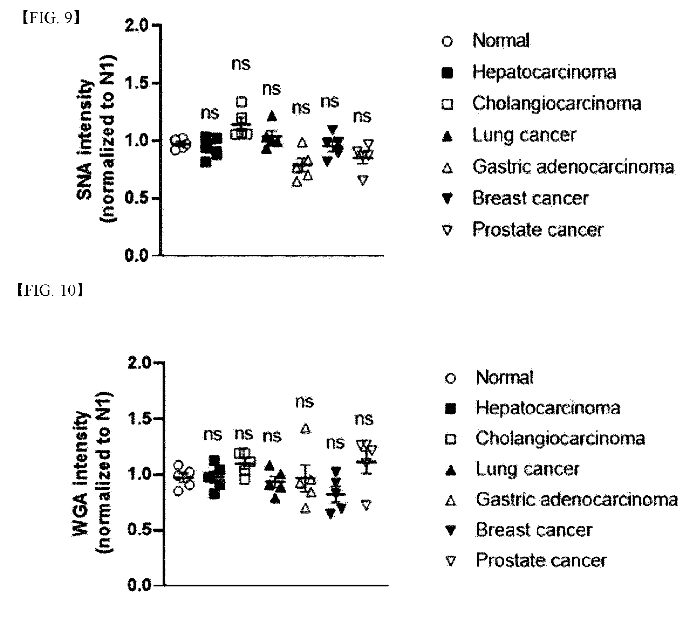

As a result, as shown in FIG. 8, it was confirmed that the level of fucosylation of exosomes was significantly higher in cholangiocarcinoma than other cancer types. In addition, as shown in FIGS. 9 and 10, it was confirmed that sialic acid and glucosamine sugars did not differ significantly from normal in all solid tumors.

That is, the level of fucosylation of serum-derived exosomes of cholangiocarcinoma patients was specifically increased in cholangiocarcinoma compared to cholecystitis, cholangitis, and other types of cancer, and in particular, the level of fucosylation of serum-derived exosomes showed an increasing pattern according to the severity of cholangiocarcinoma to provide the possibility of distinguishing the stage of cholangiocarcinoma.

Therefore, these results demonstrate that fucosylation of serum-derived exosomes (CCA-EVs) may specifically diagnose cholangiocarcinoma.

### Example 3. Analysis of sugar chain structure of patient serum-derived exosomes (CCA-EV) using HPLC

The present inventors considered the fucosylation of serum-derived exosomes as a biomarker for the diagnosis of cholangiocarcinoma, as confirmed in Example 2 above, and analyzed the sugar chain structure of serum-derived exosomes, in order to demonstrate that the fucosylation sugar chain structure of exosomes in the serum of cholangiocarcinoma patients may be applied as an important indicator for more accurately diagnosing cholangiocarcinoma.

First, the sugar chains in the exosomes were isolated and labeled with fluorescent substances, and then the fluorescence signals of the isolated sugar chains were detected using a fluorescence detector. In addition, glucose units (GU) were confirmed according to retention Time (RT) using a glucose ladder, and the type and amount of sugar chains in exosomes were confirmed based on the GU value.

At this time, the sugar chains were degraded using hydrolases and then the fluorescence signals were detected. At this time, a total of four experimental groups were used as a sialidase-alone treatment group; a sialidase and galactosidase-combined treatment group; a sialidase, galactosidase, and alpha 1-3/4 fucosidase-combined treatment group; and a sialidase, galactosidase, alpha 1-3/4 fucosidase, and N-acetylglucosaminidase-combined treatment group.

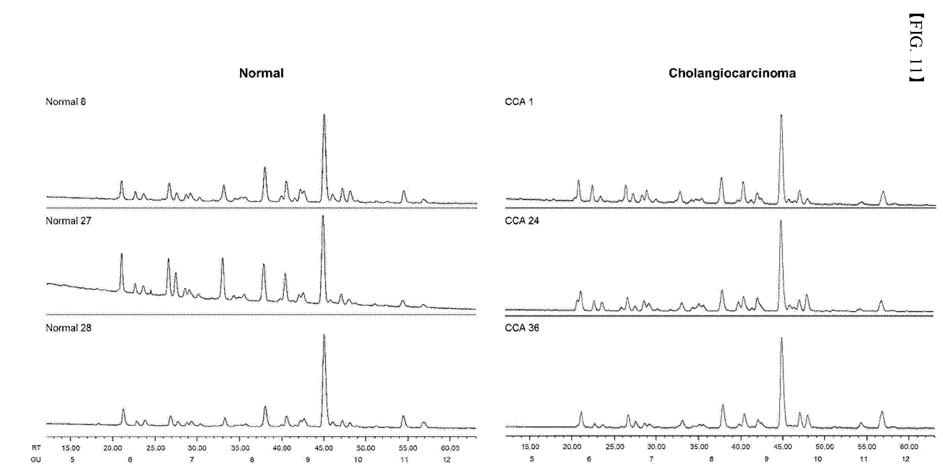

As a result, as shown in FIG. 11, normal exosomes and exosomes of cholangiocarcinoma patients showed the same type of sugar chain structure at GU between 5 and 10. In contrast, the sugar chains at GU between 10 and 11 had higher peaks in normal exosomes and lower peaks in exosomes of cholangiocarcinoma patients; and the sugar chains at GU between 11 and 12 had higher peaks in exosomes of cholangiocarcinoma patients and lower peaks in normal exosomes.

That is, it can be seen that there is a significant difference between the exosomes of cholangiocarcinoma patients (CCA) and normal exosomes (normal) in some peaks.

More specifically, based on the GU analysis results, the sugar chain structure of the GU showing the different peaks was distinguished to analyze the detailed glycan structure of the cholangiocarcinoma patient exosomes (CCA-EVs).

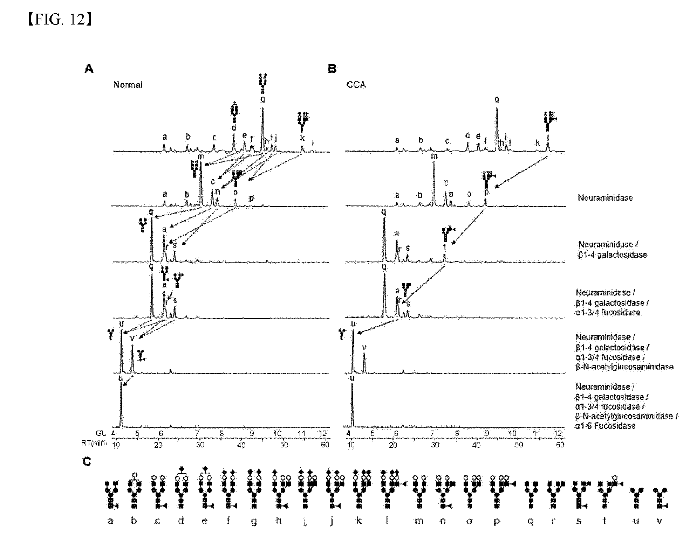

As a result, as shown in FIG. 12, when the sugar chain structure corresponding to each peak was analyzed, it was confirmed that the 2-AB glycan chromatograms of normal exosome (FIG. 12A) and cholangiocarcinoma patient exosome (FIG. 12B) samples were similar with 12 major peaks, whereas alpha 1-3/4 fucose among the sugar chain structures of the cholangiocarcinoma patient-derived exosome was significantly increased compared to the sugar chain of the normal exosome, which showed that the alpha 1-3/4 fucosylation may be used as a diagnostic marker for cholangiocarcinoma patients.

In addition, when assigning glycan structures using glucose units (GUs), bi- and tri-antenna structures with neutral glycans (peaks a to c) and sialylated glycans (peaks d to 1) were shown. Interestingly, it was found that a peak "k" was higher in normal-EVs than in CCA-EVs, while a peak "l" was higher in CCA-EVs than in normal-EVs, and it was shown that the peak k was a tri-sialylated, tri-antennary sugar chain (A3G3S3; Oxford notation name) sugar chain of the following Structural Formula 1, while the peak l was a tri-sialylated, a1-3/4 fucosylated, tri-antennary sugar chain (A3G3FS3; Oxford notation name) sugar chain of the following Structural Formula 2:

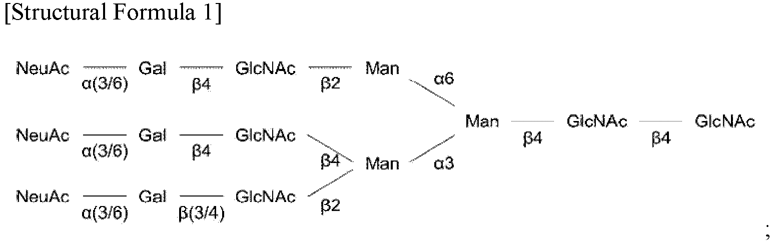

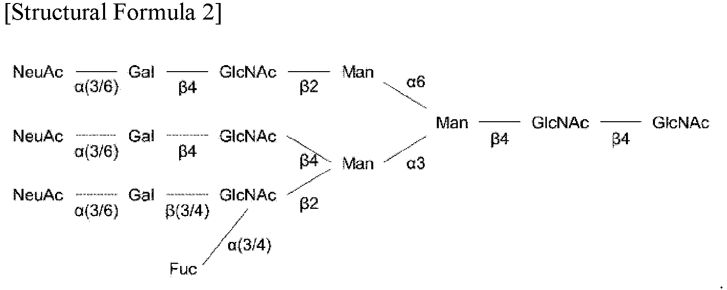

This demonstrates that these sugar chain structures, as well as the aforementioned alpha 1-3/4 fucosylation, may be used as specific diagnostic markers for cholangiocarcinoma.

### Example 4. β-haptoglobin in serum-derived exosomes (CCA-EVs) from cholangiocarcinoma patients

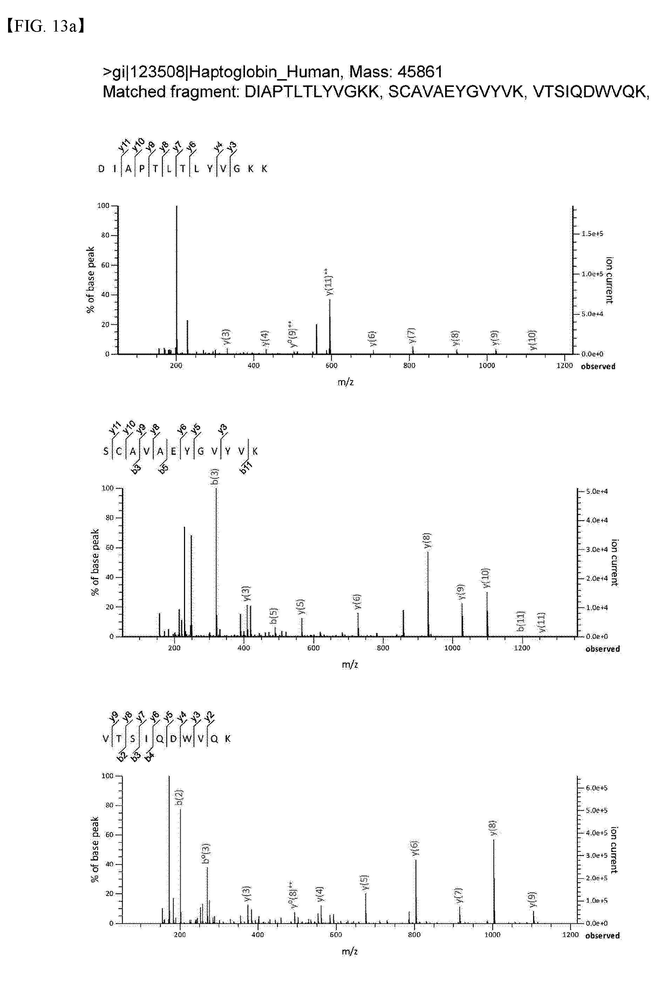

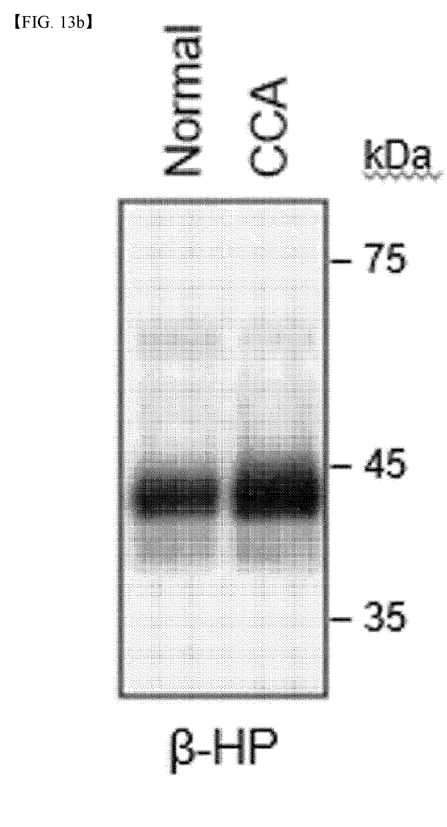

The present inventors investigated how the alpha 1-3/4 fucosylation of exosomes was increased in CCA patients. First, through proteomic analysis, it was confirmed that β-haptoglobin (β-Hp) was a protein with increased fucosyl residues (FIG. 13A). In addition, it was confirmed that similar amounts of β-Hp were present in exosomes isolated from CCA patients and a control group (FIG. 13B).

In addition, to investigate whether the abnormal glycosylation of CCA-EVs was caused by changes in Hp glycosylation, β-Hp was purified from EVs by immunoaffinity chromatography, and the glycosylation state was confirmed by AAL blotting. As a result, β-Hp of CCA-EVs was more fucosylated than that of normal EVs (FIG. 14A).

It was noted that β-Hp existed in the blood in a soluble form (s-Hp) and a membrane-bound form associated with EVs (EV-Hp). To investigate how glycosylation differed between the two forms of β-Hp and to determine which form was associated with CCA, membrane-bound β-Hp (EV-Hp) was purified from EVs and soluble β-Hp (s-Hp) was purified from EV-free serum obtained from the same patients. It was found that the reactivity of AAL and PHA-L with EV-Hp was higher than that of s-Hp, whereas SNA and WGA showed similar reactivity with both Hps (FIG. 14B).

Thus, β-Hp contained in EV showed increased fucosylation in CCA patients.

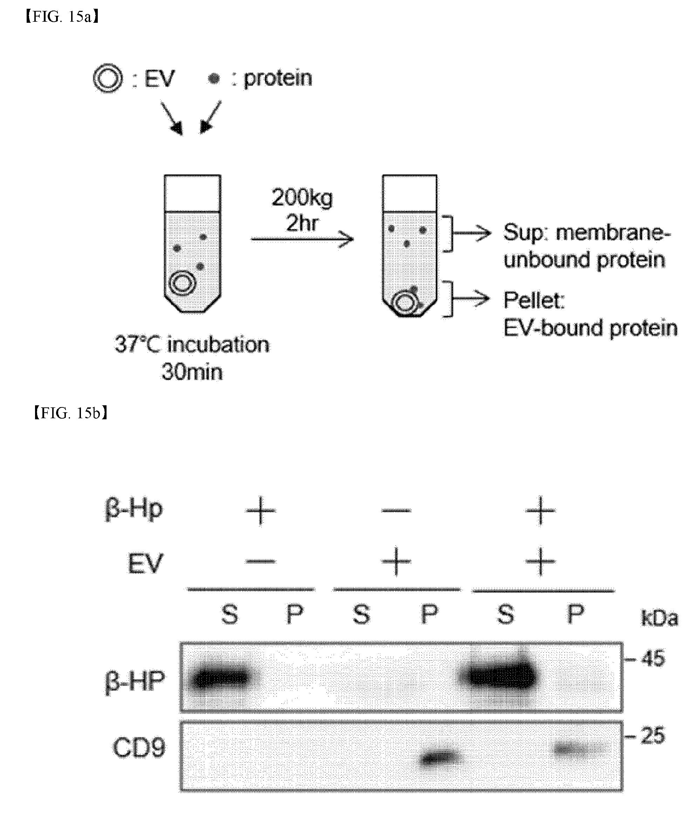

Next, the present inventors intended to investigate how β-Hp, known as a secreted soluble protein, was associated with EVs. EVs serve as a membrane platform for binding of soluble proteins while circulating in the blood, and to investigate whether this was a case for β-Hp, isolated EVs were incubated with purified β-Hp and it was confirmed that β-Hp was not collected to the membrane (FIGS. 15A and 15B).

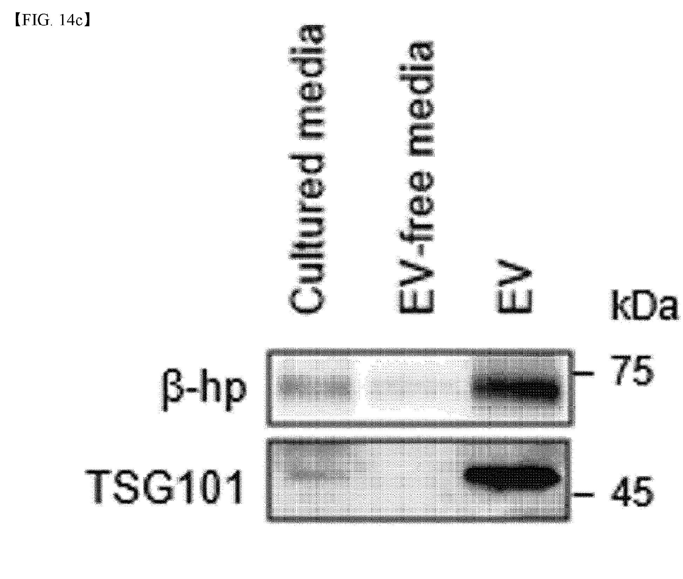

Alternatively, it was investigated whether β-Hp was secreted into cells via EVs. As a result, in cells expressing GFP-tagged β-Hp, more GFP signals were detected in EVs isolated from the culture medium than in the EV-free culture medium (FIG. 14C).

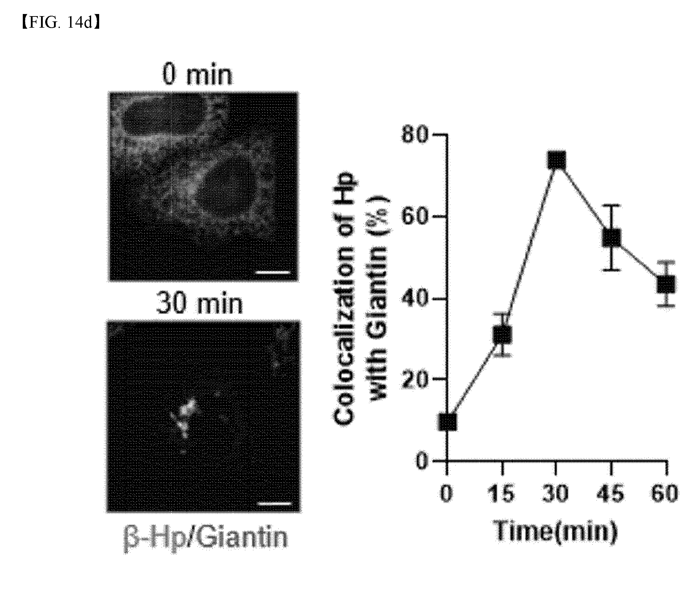

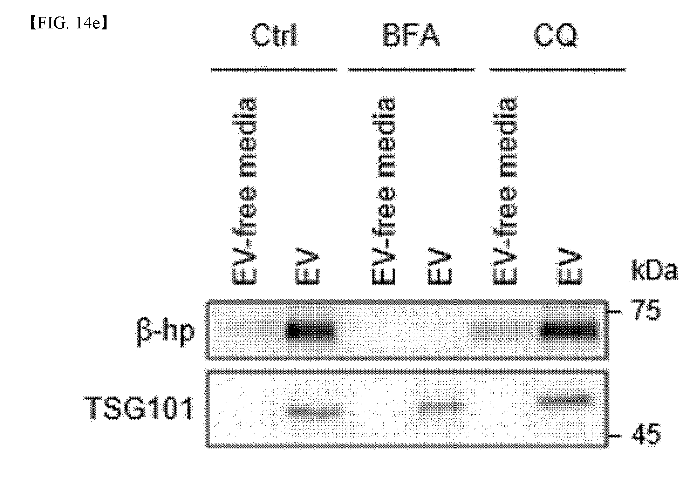

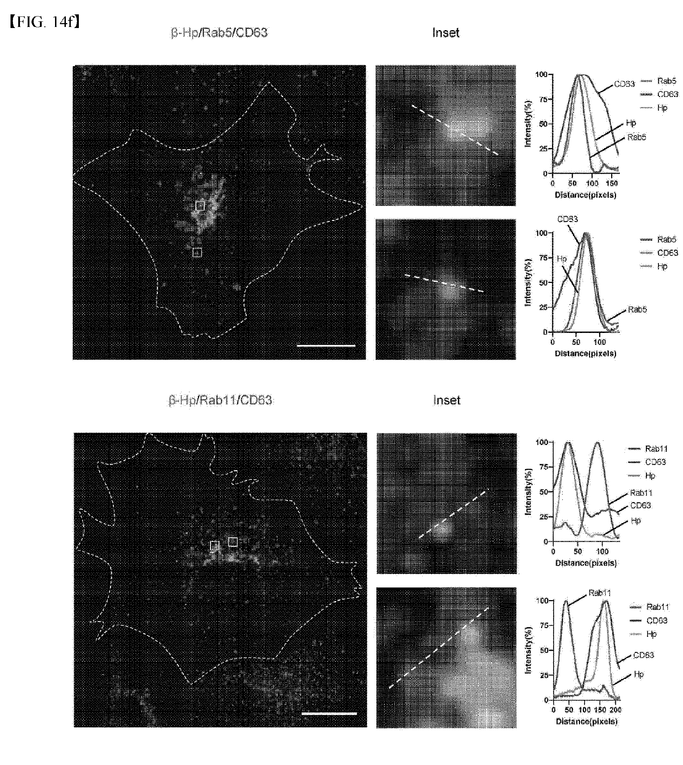

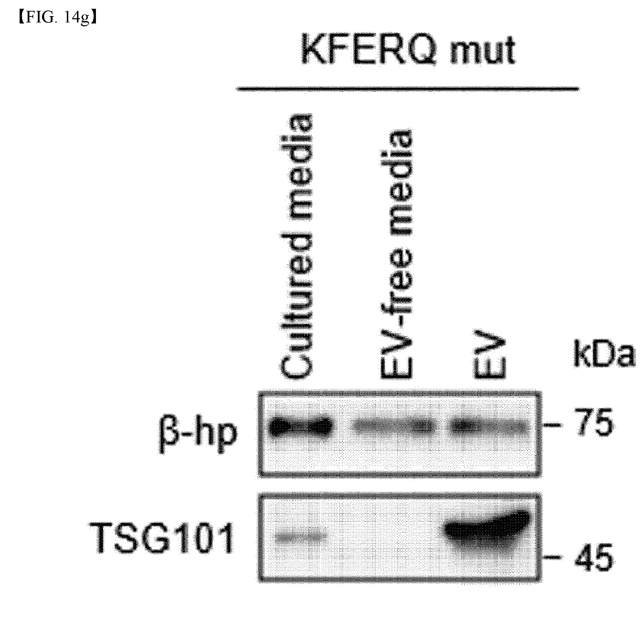

Furthermore, the present inventors confirmed that newly synthesized β-Hp was transported via a conventional secretory pathway by using real-time tracking of protein secretion using RUSH (FIG. 14D). EV-mediated secretion of β-Hp was inhibited by destruction of the Golgi complex using brefeldin-A (BFA), but not inhibited by inhibition of the endo/lysosomal pathway using chloroquine (FIG. 14E). It was confirmed that this inhibition was not induced by reduced EV biogenesis as determined by TSG101 levels (FIG. 14E). β-Hp released from the Golgi was transported through RabS-positive endosomes colocalized with CD63, but was not transported through Rab11-positive endosomes (FIG. 14F). As a soluble cargo was transported into EVs via the KFERQ motif, it was confirmed that β-Hp contained a KFERQ-like motif by performing a motif finder search, and it was confirmed that when this motif was disturbed by mutation, the amount of β-Hp secreted in a soluble form was increased (FIG. 14G). Accordingly, it was demonstrated that β-Hp was secreted from cells via EVs.

### Example 5. Tumor growth promotion by fucosylated EVs derived from CCA cells

To investigate how EV fucosylation was increased in CCA patients, the present inventors evaluated gene expression profiles in a GSE26566 dataset and found that several genes showed changed expression in CCA compared to surrounding liver and normal intrahepatic bile ducts. Among them, fucosyltransferase-3 and -4 (FUT-3/-4), a gene encoding a factor required for alpha 1-3/4 fucosylation, was highly expressed in CCA (FIG. 16A).

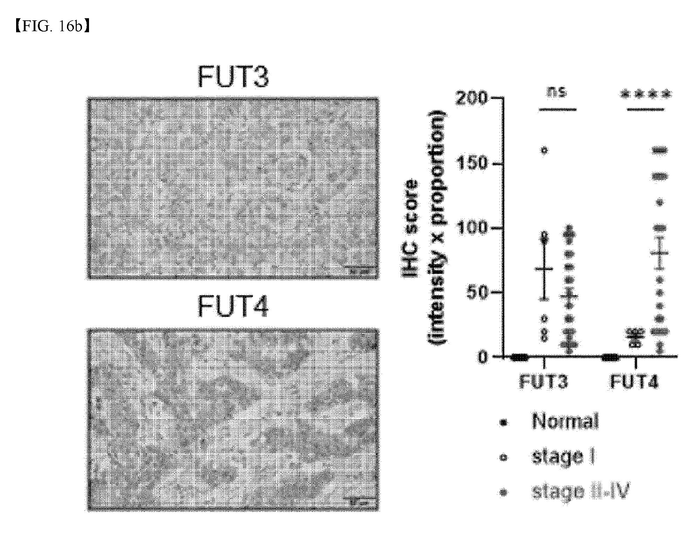

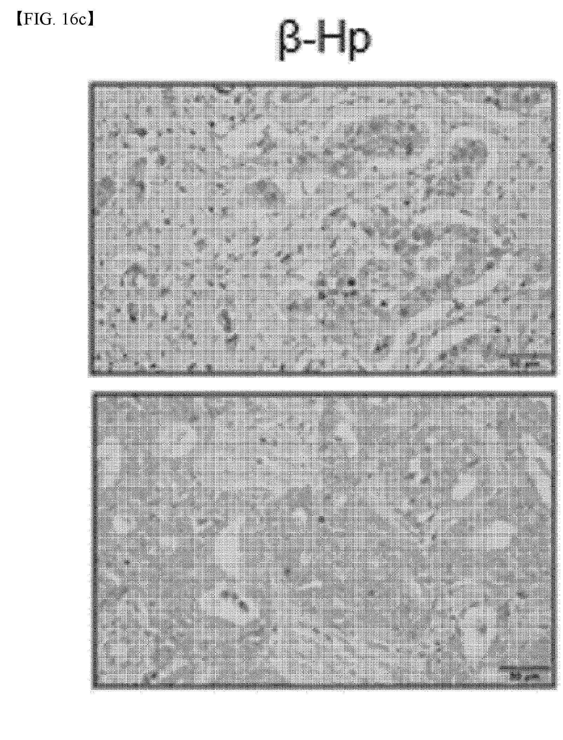

FUT-3/-4 expressions were further investigated using immunohistochemistry. As a result, it was confirmed that FUT3 was highly expressed in early cancer, whereas FUT4 expression gradually increased as the stage of the disease progressed (FIG. 16B). In addition, β-Hp expression was increased in CCA tissue (FIG. 16C).

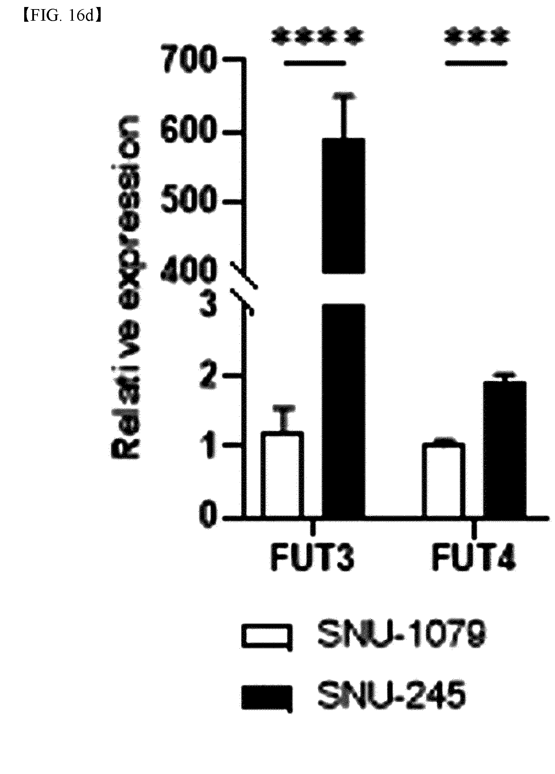

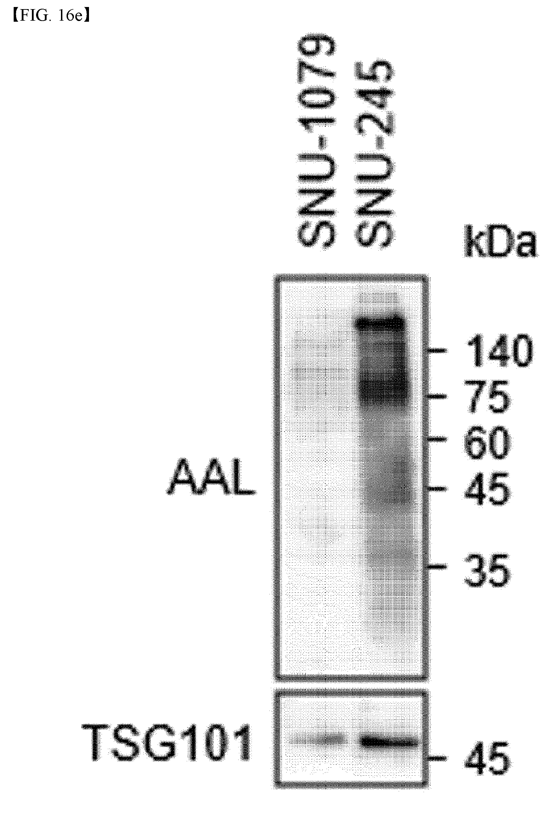

To determine the effect of tumor location on gene expression profiles, FUT-3 and FUT-4 expressions were quantified in two CCA cell lines which were similarly differentiated, but derived from different locations. As a result, FUT-3/-4 were expressed more in an extrahepatic cholangiocarcinoma (EHCC)-derived SNU-245 cell line than in an intrahepatic cholangiocarcinoma (IHCC)-derived SNU-1079 cell line (FIG. 16D). In addition, β-Hp of SNU-245 secreted EVs showed greater fucosylation than β-Hp of SNU-1079 secreted EVs (FIG. 16E).

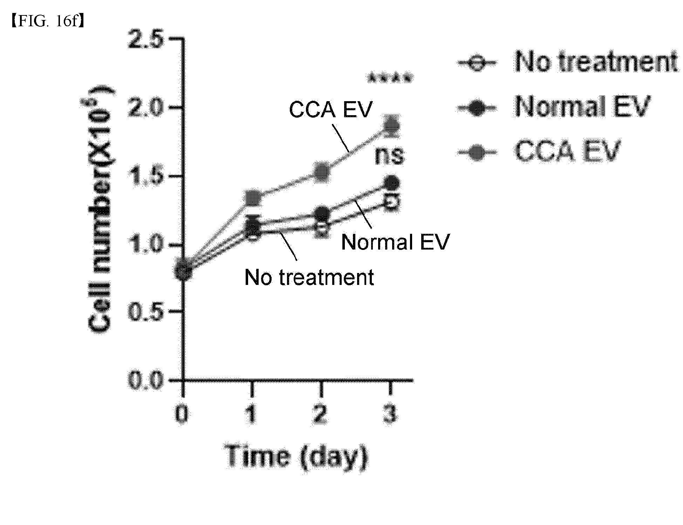

In addition, the present inventors investigated the effect of fucosylated EVs on tumor growth. SNU-1079 cells treated with fucosylated EVs isolated from CCA patients showed growth promotion, whereas normal EVs had no such effect (FIG. 16F).

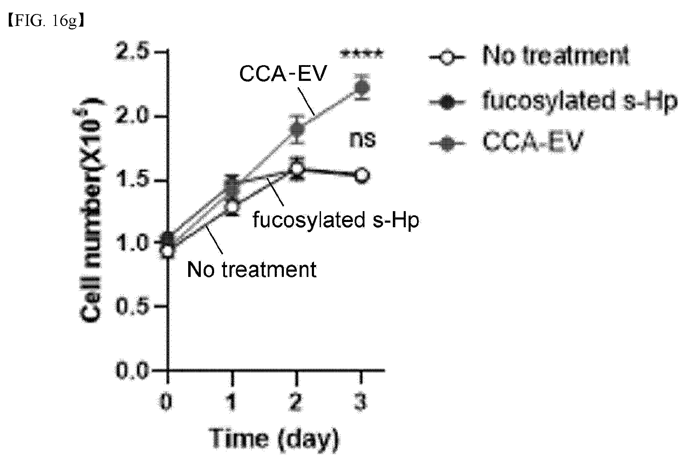

It was confirmed that cancer cell growth was not induced by the soluble form of fucosylated Hp, which indicated that this function was specific for EV-Hp (FIG. 16G). These results demonstrated that fucosylated EVs in the sera of CCA patients were derived from pathologically heterogeneous CCA cells and promoted tumor growth.

That is, it was shown that alpha 1-3/4 fucosylation of β-haptoglobin (β-Hp) contained in EVs was specifically associated with tumor stage, location, treatment response, and recurrence of CCA, but was not associated with other solid tumors. Interestingly, glycosylation of blood β-Hp, which was not involved in EV, was not altered in CCA patients. These results suggest that alpha 1-3/4 fucosylated EVs in the blood have diagnostic and prognostic values in CCA.

In conclusion, it was confirmed that it is possible to diagnose and predict the prognosis of cholangiocarcinoma by using a biomarker capable of detecting exosome-specific carbohydrate sugar chain changes derived (isolated, extracted) from the serum of cholangiocarcinoma patients of the present disclosure.

As described above, specific parts of the present disclosure have been described in detail, and it will be apparent to those skilled in the art that these specific techniques are merely preferred embodiments, and the scope of the present disclosure is not limited thereto. Therefore, the substantial scope of the present disclosure will be defined by the appended claims and their equivalents.

## Claims

1. A biomarker composition for diagnosing or predicting prognosis of cholangiocarcinoma, comprising fucosylation of exosomes.

2. A composition for diagnosing or predicting prognosis of cholangiocarcinoma, comprising an agent capable of detecting fucosylation of exosomes.

3. The composition for diagnosing or predicting the prognosis of cholangiocarcinoma of claim 2, wherein the fucosylation of the exosomes is fucosylation of sugar chains of an exosome surface protein.

4. The composition for diagnosing or predicting the prognosis of cholangiocarcinoma of claim 2, wherein the agent capable of detecting the fucosylation is lectin.

5. The composition for diagnosing or predicting the prognosis of cholangiocarcinoma of claim 4, wherein the lectin is at least one selected from the group consisting of aleuria aurantia lectin (AAL), aspergillus oryzae lectin (AOL), ulex europaeus agglutinin I (UEA I), and lotus tetragonolobus lectin (LTL).

6. A kit for diagnosing or predicting prognosis of cholangiocarcinoma, comprising the composition of any one of claims 2 to 5.

7. A method for diagnosing and treating cholangiocarcinoma, comprising the following steps:
(a) isolating exosomes derived from a subject;
(b) measuring a level of fucosylation of the exosomes by treating the isolated exosomes with an agent capable of detecting the fucosylation; and
(c) administering a cholangiocarcinoma therapeutic agent to a subject diagnosed with the cholangiocarcinoma in step (b).

8. The method for diagnosing and treating cholangiocarcinoma of claim 7, wherein when the level of fucosylation of the exosomes in step (b) is increased compared to a normal control sample, it is determined as the cholangiocarcinoma or to have a poor prognosis.

9. The method for diagnosing and treating cholangiocarcinoma of claim 7, wherein the agent capable of detecting the fucosylation in step (b) is lectin.

10. The method for diagnosing and treating cholangiocarcinoma of claim 9, wherein the lectin is at least one selected from the group consisting of aleuria aurantia lectin (AAL), aspergillus oryzae lectin (AOL), ulex europaeus agglutinin I (UEA I), and lotus tetragonolobus lectin (LTL).

11. The method for diagnosing and treating cholangiocarcinoma of claim 7, wherein the exosomes are isolated from at least one biological sample selected from the group consisting of blood, serum, plasma, saliva, sputum, and urine.

12. The method for diagnosing and treating cholangiocarcinoma of claim 7, wherein the cholangiocarcinoma therapeutic agent is at least one anticancer agent selected from the group consisting of 5-fluorouracil, gemcitabine, cisplatin, capecitabine, oxaliplatin, doxorubicin, carboplatin, cyclophosphamide, ifosfamide, nidran, bleomycin, mitomycin C, cytarabine, trimetrexate, methotrexate, etoposide, vinblastine, vinorelbine, alimta, altretamine, procarbazine, taxol, taxotere, lucitanib, erdafitinib, herceptin, pertuzumab, topotecan, pemigatinib, infigratinib, and irinotecan.

13. A biomarker composition for diagnosing or predicting prognosis of cholangiocarcinoma, comprising alpha 1-3/4 fucose sugar chains of exosomes.

14. A composition for diagnosing or predicting prognosis of cholangiocarcinoma, comprising an agent capable of detecting alpha 1-3/4 fucose sugar chains of exosomes as an active ingredient.

15. The composition for diagnosing or predicting the prognosis of cholangiocarcinoma of claim 14, wherein the alpha 1-3/4 fucose sugar chains of the exosomes are sugar chains of exosome surface proteins.

16. The composition for diagnosing or predicting the prognosis of cholangiocarcinoma of claim 15, wherein the sugar chain is any one sugar chain of the following sugar chains:
tri-sialylated, tri-antennary sugar chain (A3G3S3) of the following Structural Formula 1 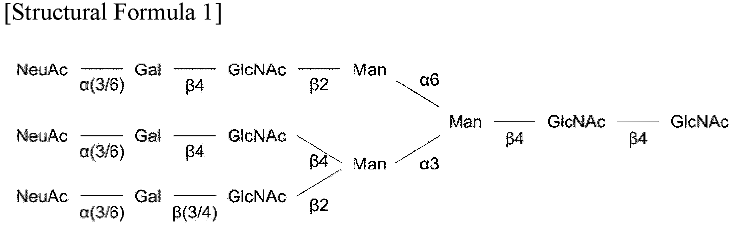 ; or
tri-sialylated, a1-3/4 fucosylated, tri-antennary sugar chain (A3G3FS3) of the following Structural Formula 2

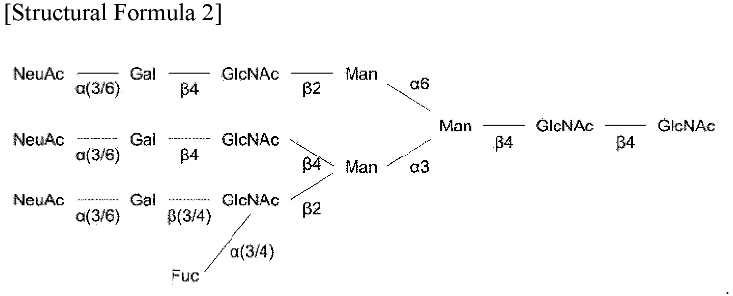

17. The composition for diagnosing or predicting the prognosis of cholangiocarcinoma of claim 15, wherein the exosome surface protein is β-haptoglobin.

18. The composition for diagnosing or predicting the prognosis of cholangiocarcinoma of claim 14, wherein the alpha 1-3/4 fucose sugar chain is detected by at least one selected from the group consisting of high-performance liquid chromatography (HPLC), capillary electrophoresis, isoelectric focusing, mass spectrometry, porous graphite chromatography, and SDS-PAGE.

19. A kit for diagnosing or predicting prognosis of cholangiocarcinoma, comprising the composition of any one of claims 13 to 18.

20. A method for diagnosing and treating cholangiocarcinoma, comprising the following steps:
(a) isolating sugar chains from a subject-derived exosome;
(b) measuring a level of alpha 1-3/4 fucose sugar chains among the isolated sugar chains of the exosome; and
(c) administering a cholangiocarcinoma therapeutic agent to a subject diagnosed with the cholangiocarcinoma in step (b).

21. The method for diagnosing and treating cholangiocarcinoma of claim 20, wherein when the level of the alpha 1-3/4 fucose sugar chain of the exosome of step (b) is increased compared to a normal control sample, it is determined as the cholangiocarcinoma.

22. The method for diagnosing and treating cholangiocarcinoma of claim 21, wherein the alpha 1-3/4 fucose sugar chain of the exosome is a sugar chain of an exosome surface protein.

23. The method for diagnosing and treating cholangiocarcinoma of claim 22, wherein the exosome surface protein is β-haptoglobin.

24. A composition for diagnosing or predicting prognosis of cholangiocarcinoma, comprising an agent capable of detecting fucosylation of an exosome membrane-bound form, haptoglobin.

25. A kit for diagnosing or predicting prognosis of cholangiocarcinoma, comprising the composition of claim 24.

26. A biomarker composition for diagnosing or predicting prognosis of cholangiocarcinoma, comprising any one sugar chain of the following sugar chains:
tri-sialylated, tri-antennary sugar chain (A3G3S3) of the following Structural Formula 1 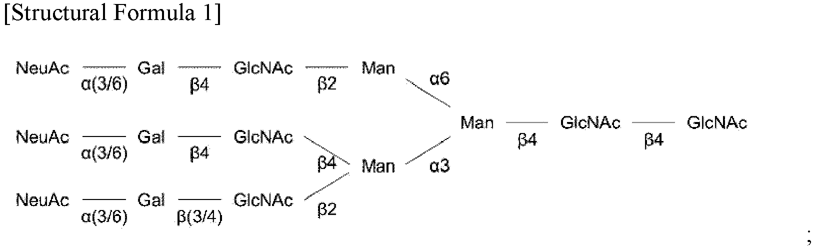 or
tri-sialylated, a1-3/4 fucosylated, tri-antennary sugar chain (A3G3FS3) of the following Structural Formula 2

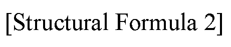

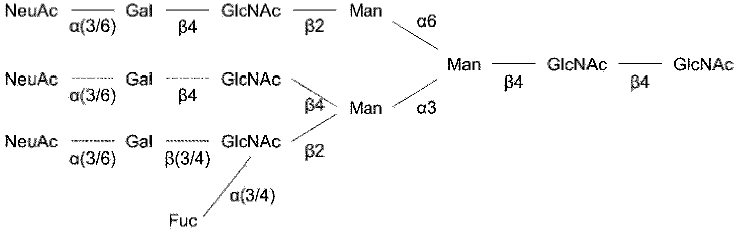

27. The biomarker composition for diagnosing or predicting the prognosis of cholangiocarcinoma of claim 26, wherein the sugar chain is a sugar chain of an exosome surface protein.

28. A composition for diagnosing or predicting prognosis of cholangiocarcinoma, comprising an agent capable of detecting any one sugar chain of the following sugar chains:
tri-sialylated, tri-antennary sugar chain (A3G3S3) of the following Structural Formula 1 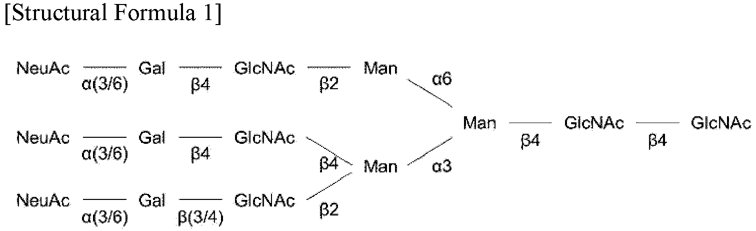 ; or
tri-sialylated, a1-3/4 fucosylated, tri-antennary sugar chain (A3G3FS3) of the following Structural Formula 2

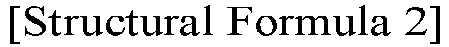

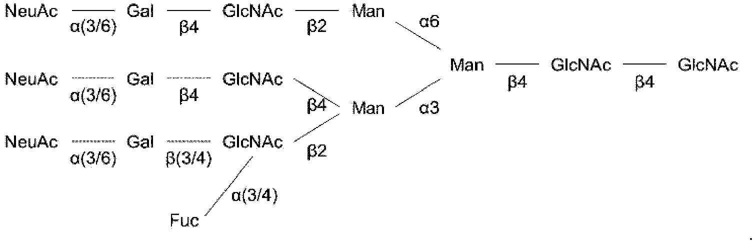

29. A kit for diagnosing or predicting prognosis of cholangiocarcinoma, comprising the composition of any one of claims 26 to 28.

30. A method for diagnosing and treating cholangiocarcinoma comprising:
(a) isolating sugar chains from a subject-derived exosome;
(b) detecting tri-sialylated, tri-antennary sugar chain (A3G3S3) of the following Structural Formula 1; or tri-sialylated, a1-3/4 fucosylated, tri-antennary sugar chain (A3G3FS3) of the following Structural Formula 2 among the isolated sugar chains of the exosomes; and
(c) administering a cholangiocarcinoma therapeutic agent to a subject diagnosed with the cholangiocarcinoma in step (b). 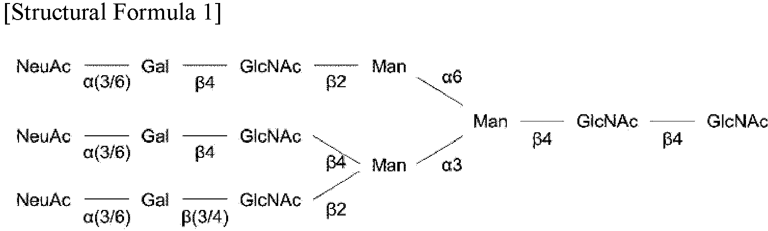 ; or

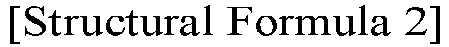

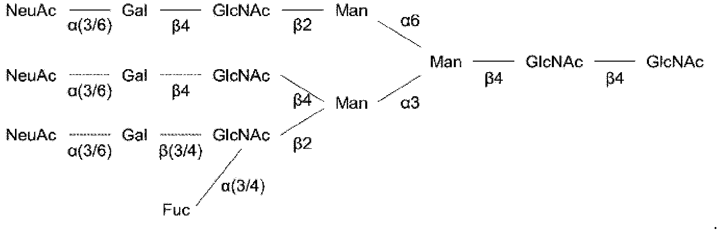

31. The method for diagnosing and treating cholangiocarcinoma of claim 30, wherein the sugar chain is a sugar chain of an exosome surface protein.

32. The method for diagnosing and treating cholangiocarcinoma of claim 30, wherein the exosome sugar chains of step (b) are detected through glucose units (GU) by high-performance liquid chromatography (HPLC).

33. The method for diagnosing and treating cholangiocarcinoma of claim 30, wherein when the tri-sialylated, a1-3/4 fucosylated, tri-antennary sugar chain (A3G3FS3) sugar chain in step (b) is increased compared to a normal control sample, it is determined as the cholangiocarcinoma or to have a poor prognosis.

34. The method for diagnosing and treating cholangiocarcinoma of claim 30, wherein when the tri-sialylated, tri-antennary sugar chain (A3G3S3) sugar chain in step (b) is decreased compared to the normal control sample, it is determined as the cholangiocarcinoma or to have a poor prognosis.
